# EUROPEAN PATENT SPECIFICATION

(11) **EP 4 504 967 B1**
(45) Date of publication and mention of the grant of the patent: **02.07.2025**
(21) Application number: 24723984.1
(22) Date of filing: 29.04.2024
(51) Int. Cl.: C12Q 1/682, C12Q 1/6853

(54) **METHOD FOR DETECTING TARGET NUCLEIC ACID MOLECULES**
VERFAHREN ZUM NACHWEIS VON ZIELNUKLEINSÄUREMOLEKÜLEN
PROCÉDÉ DE DÉTECTION DE MOLÉCULES D'ACIDE NUCLÉIQUE CIBLES

(30) Priority: 03.05.2023 SE 2350529
(43) Date of publication of application: 12.02.2025
(73) Proprietor: Readily AB, 750 02 Uppsala (SE)
(72) Inventor: LÖF, Liza, 759 91 Uppsala (SE); MOLIN, Magnus, 756 56 Uppsala (SE)
(74) Representative: Barker Brettell Sweden AB
(86) International application number: PCT/SE2024/050412
(87) International publication number: WO 2024/228656

(56) References cited:
- WO-A1-2022/194612
- US-A1- 2023 115 903
- CARL-MAGNUS CLAUSSON ET AL: "Compaction of rolling circle amplification products increases signal integrity and signal-to-noise ratio", SCIENTIFIC REPORTS, vol. 5, 23 July 2015 (2015-07-23), pages 12317, XP055305777, DOI: 10.1038/srep12317
- SEPEHRI SOBHAN ET AL: "Characterization of Binding of Magnetic Nanoparticles to Rolling Circle Amplification Products by Turn-On Magnetic Assay", BIOSENSORS, vol. 9, no. 3, 17 September 2019 (2019-09-17), pages 109, XP055824371, DOI: 10.3390/bios9030109
- CONGLI TANG: "Naked-Eye Detection of Food-Borne Pathogens Using Multiplex Hyperbranched Rolling Circle Amplification and Magnetic Particles", BIOSENSORS, vol. 12, no. 1075, 25 November 2022 (2022-11-25), CH, pages 1 - 11, XP093161395, ISSN: 2079-6374, DOI: 10.3390/bios12121075

## Description

### TECHNICAL FIELD

The present invention generally relates to detection of target nucleic acid molecules, and in particular to detection of such target nucleic acid molecules using padlock probes and rolling circle amplification.

### BACKGROUND

The detection of target nucleic acid molecules has applications in many different fields, including notably clinically, for personalized medicine and in the diagnosis, prognosis and/or treatment of diseases, such as cancer, infectious diseases and inherited or genetic disorders, as well as in research and biosecurity.

Target nucleic acid molecules may be detected using labelled hybridization probes, but hybridization probes have relatively high detection limit, and cannot readily be used to discriminate between similar nucleic acid sequences. To increase sensitivity, target nucleic acid molecules are typically amplified, to increase the amount of target nucleic acid sequence available for detection. Any of a variety of techniques known in the art may be used for the amplification, including rolling circle amplification (RCA).

RCA utilizes a strand displacement polymerase enzyme, and requires a circular amplification template. Amplification of the circular template provides a concatenated RCA product (RCP) comprising multiple copies of a sequence complementary to that of the amplification template. Such a concatemer typically forms a ball or "blob", which may be visualized and detected, and thus RCA-based assays have been adopted for the detection of nucleic acid molecules.

The specificity of nucleic acid molecule detection may be improved by the use of probes requiring dual recognition, or two binding sites for a target nucleic acid molecule, such as padlock probes. Padlock probes are linear oligonucleotides with two separate target-complementary binding regions, connected by an intervening "backbone" region. When the padlock probe has bound (hybridized) to its target nucleic acid sequence, the ends of the padlock probe may be ligated together to circularize the padlock probe. The circularized padlock probe may then be used as the template for a RCA reaction, and the RCP may be detected. The usage of padlock probes in detection of target nucleic acid molecules was initially described in WO 97/09069.

WO 2018/109206 relates to multiplexed methods of detecting an analyte in a sample using two or more padlock probes each specific to a different target sequence. The target sequence is either part of an analyte or indicative of the presence of an analyte in the sample. Each padlock probe comprises an analyte-specific reporter sequence, and either a restriction cleavage site located 3' of the analyte-specific reporter sequence or a first amplification primer binding site for an amplification reaction.

Padlock probes and RCA have been suggested as detection of nucleic acid molecules of pathogens, such as viruses or bacteria, such as in WO 2021/202786 and WO 2022/026891.

Clausson et al., Compaction of rolling circle amplification products increases signal integrity and signal-to-noise ratio, Scientific reports, 5: 12317 (2015) discloses the introduction of a cross-hybridizing DNA oligonucleotide during rolling circle amplification to obtain smaller fluorophore-labeled RCPs. The reduced size of the RCPS increases the local concentration of fluorophores and thereby the signal intensity increases together with the signal-to-noise ratio.

Tang, Naked-eye detection of food-borne pathogens using multiplex hyperbranched rolling circle amplification and magnetic particles, Biosensors, 12(5): 1-11 (2022) discloses an assay for detection of pathogens. The assay uses a pair of universal primers for identifying target genes per reach tube. Ring padlock probes and corresponding universal primers start hyperbranched rolling circle amplification (HRCS) under the action of the polymerase to obtain branched chain amplification products, which are entangled with magnetic particles to form aggregated magnetic particle clusters.

A limitation of the prior art technology is that RCA needs to be run for a relative long period of time in order to produce RCPs of sufficient length in order to form a ball or "blob", which may be visualized and detected.

There is, thus, a need for a technology that speeds up the detection of target nucleic acid molecules.

### SUMMARY

It is a general objective to enable detection of target nucleic acid molecules in a shorter period of time as compared to prior art protocols based on padlock probes and rolling circle amplification.

This and other objectives are met by embodiments as disclosed herein.

The present invention is defined in the independent claims. Further embodiments of the invention are defined in the dependent claims.

An aspect of the invention relates to a method for detecting at least one target nucleic acid molecule in a sample. The method comprises contacting the sample with padlock probes comprising at their 5' and 3' ends target-binding regions complementary to probe-binding regions in the at least one target nucleic acid molecule. The method also comprises joining the 5' and 3' ends of the padlock probes while the target-binding regions are hybridized to the probe-binding regions to form circular padlock probes. The method further comprises rolling circle amplifying the circular padlock probes with labelled amplification primers comprising a detectable label and a probe-binding region complementary to a primer-binding region of the padlock probes to generate labelled rolling circle products. The method further comprises contacting the labelled rolling circle products with oligonucleotides comprising a plurality of binding regions having a nucleic acid sequence corresponding to at least a portion of the padlock probes outside of the primer-binding region to form an agglutinate of labelled complexes between the oligonucleotides and the labelled rolling circle products. The method additionally comprises detecting the agglutinate of the labelled complexes to detect the at least one target nucleic acid molecule.

Another aspect of the invention relates to kit for detecting at least one target nucleic acid molecule in a sample. The kit comprises padlock probes comprising at their 5' and 3' ends target-binding regions complementary to probe-binding regions in at least one target nucleic acid molecule. The kit also comprises labelled amplification primers comprising a detectable label and a probe-binding region complementary to a primer-binding region of the padlock probes. The kit further comprises oligonucleotides comprising a plurality of binding regions having a nucleic acid sequence corresponding to at least a portion of the padlock probes outside of the primer-binding region.

A further aspect of the invention relates to a microfluidic chip comprising a sample input configured to receive a sample comprising at least one target nucleic acid molecule and a microfluidic channel in fluid connection with the sample input. The microfluidic channel comprises a reagent section comprising i) padlock probes comprising at their 5' and 3' ends target-binding regions complementary to probe-binding regions in the at least one target nucleic acid molecule, ii) a ligase adapted to ligate the 5' and 3' ends of the padlock probes while the target-binding regions are hybridized to the probe-binding regions to form circular padlock probes, iii) labelled amplification primers comprising a detectable label and a probe-binding region complementary to a primer-binding region of the padlock probes, and iv) a polymerase adapted to rolling circle amplify the circular padlock probes with the labelled amplification primers to generate labelled rolling circle products. The microfluidic channel also comprises a detection section comprising oligonucleotides comprising a plurality of binding regions having a nucleic acid sequence corresponding to at least a portion of the padlock probes outside of the primer-binding region. The microfluidic chip also comprises a detection window aligned with at least a portion of the detection section and arranged to enable visual access to the at least a portion of the detection section.

The agglutinate-promoting oligonucleotides contacted with the labelled rolling circle products (RCPs) promote formation of a detectable ball or agglutinate even for comparatively short RCPs, thereby only requiring a short duration of rolling circle amplification to get a detectable signal. Experimental data as presented herein indicate that the RCA can be reduced by at least a factor of four and still producing a more easily detectable signal as compared to prior art protocols lacking the usage of agglutinate-promoting oligonucleotides.

### BRIEF DESCRIPTION OF THE DRAWINGS

The embodiments, together with further objects and advantages thereof, may best be understood by making reference to the following description taken together with the accompanying drawings, in which:
Fig. 1 schematically illustrates a padlock probe hybridized to a target nucleic acid molecule according to an embodiment;
Fig. 2 schematically illustrates a circular padlock probe hybridized to a target nucleic acid molecule according to an embodiment;
Fig. 3 schematically illustrates a labelled amplification primer hybridized to a circular padlock probe according to an embodiment;
Fig. 4 schematically illustrates rolling circle amplification of a circular padlock probe according to an embodiment;
Fig. 5 schematically illustrates a labelled rolling circle product according to an embodiment;
Fig. 6 schematically illustrates a labelled complex according to an embodiment;
Fig. 7 schematically illustrates a labelled complex according to another embodiment;
Fig. 8 schematically illustrates an agglutinate of labelled complexes according to an embodiment;
Fig. 9 schematically illustrates labelled amplification primers hybridized to a circular padlock probe according to an embodiment;
Fig. 10 schematically illustrates padlock probes hybridized to a target nucleic acid molecule according to an embodiment;
Fig. 11 schematically illustrates labelled amplification primers hybridized to circular padlock probes according to an embodiment;
Fig. 12 schematically illustrates labelled amplification primers hybridized to circular padlock probes according to another embodiment;
Fig. 13 schematically illustrates padlock probes hybridized to target nucleic acid molecules according to an embodiment;
Fig. 14 schematically illustrates labelled amplification primers hybridized to circular padlock probes according to an embodiment;
Fig. 15 schematically illustrates rolling circle amplifying of circular padlock probes according to an embodiment;
Fig. 16 schematically illustrates labelled complexes according to an embodiment;
Fig. 17 schematically illustrates a padlock probe hybridized to target nucleic acid molecules according to an embodiment;
Fig. 18 is a flow chart illustrating an embodiment of a method of detecting a target nucleic acid molecule according to an embodiment;
Fig. 19 is a flow chart illustrating an additional, optional step of the method shown in Fig. 18 according to an embodiment;
Fig. 20 is a flow chart illustrating an embodiment of the removing step in Fig. 19;
Figs. 21A and 21B illustrate a microfluidic chip in an upper view and a cross-sectional view according to an embodiment;
Figs. 22A and 22B illustrate a microfluidic chip in an upper view and a cross-sectional view according to another embodiment;
Figs. 23A to 23C illustrate reaction tubes after 10 minutes (23A), 25 minutes (23B) or 40 minutes (23C) of rolling circle amplification without any pre-made oligonucleotides;
Fig. 24 illustrate reaction tubes after 10 minutes of rolling circle amplification with pre-made oligonucleotides; and
Fig. 25 illustrate reaction tubes with pre-made oligonucleotides with correct or incorrect sequence.

### DETAILED DESCRIPTION

The present invention generally relates to detection of target nucleic acid molecules, and in particular to detection of such target nucleic acid molecules using padlock probes and rolling circle amplification.

Padlock probes and rolling circle amplification (RCA) can be used to detect the presence of target nucleic acid molecules in a sample at a high specificity since the padlock probes require dual recognition and ligation to form a circular padlock probe that can be amplified by RCA into an RCA product (RCP), which is a concatemeric product comprising multiple repeats of a complementary copy of the circularized padlock probe. In the art, such an RCP can be detected using labelled amplification primers during RCA so that the resulting RCPs will incorporate the label. If the RCPs have sufficient length, i.e., contain a sufficient high number of complementary copies of the circular padlock probe, the RCPs will become entangled with each other forming a labelled ball that could, due to the presence of the labels, be detected. In the art, the RCA must therefore be run for a comparatively long period of time in order to produce RCPs of sufficient length in order to form the ball or "blob", which may be visualized and detected. As a consequence, the RCA reaction time constitutes the major portion of the total time for detecting target nucleic acid molecules in a sample in the prior art.

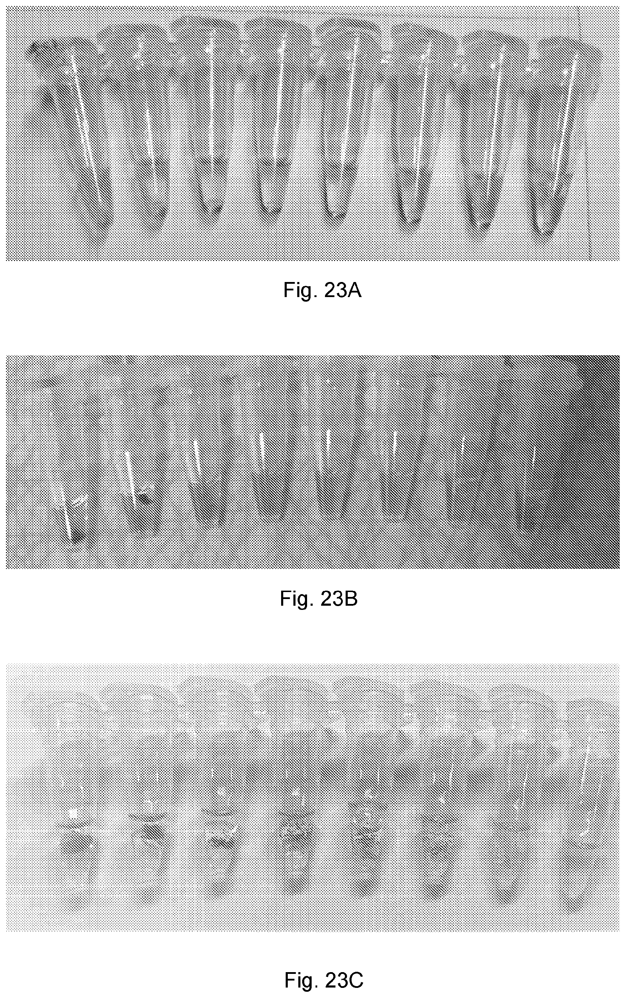

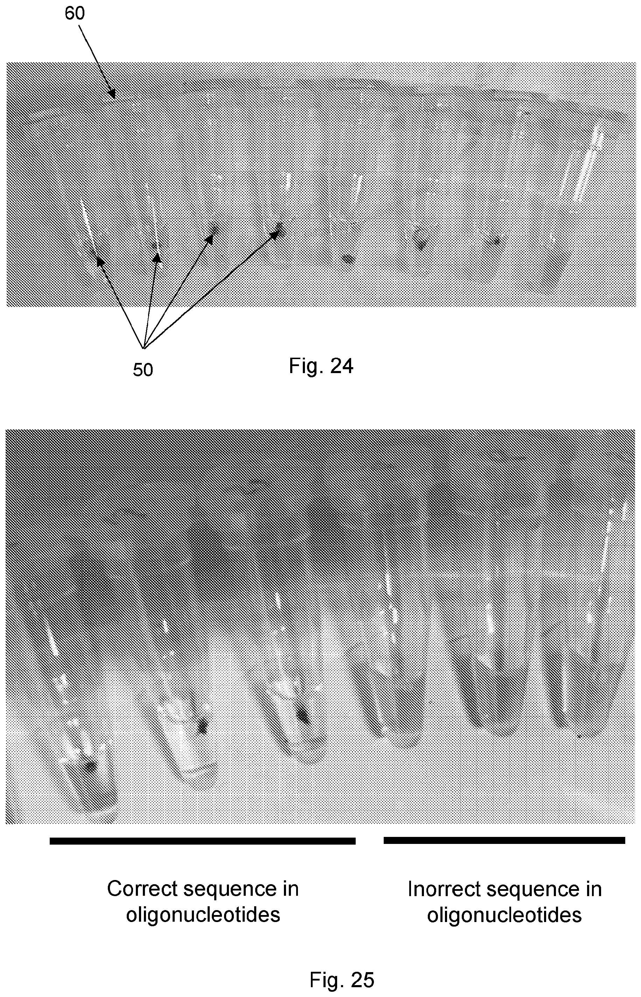

The present invention significantly speeds up such detection by merely requiring comparatively shorter RCPs, and thereby shorter RCA reaction times, and still being able to detect the labelled RCPs. This is possible according to the invention by using oligonucleotides comprising a plurality of binding regions, to which the labelled RCPs can bind. The oligonucleotides thereby promote entanglement of also shorter labelled RCPs to form a detectable ball or "blob", i.e., an agglutinate or aggregate, at a significant shorter period of time as compared to prior art techniques. This can be seen by comparing Fig. 24 showing detectable agglutinates or aggregates of labelled RCPs bound to oligonucleotides according to the invention following 10 min of RCA and Figs. 23A-23C showing prior art, i.e., no oligonucleotides, after 10 min (Fig. 23A), 25 min (Fig. 23B) and 40 min (Fig. 23C) of RCA. Hence, the present invention provides more easily detectable labelled complexes already after merely 10 min of RCA as compared to the prior art with 40 min of RCA.

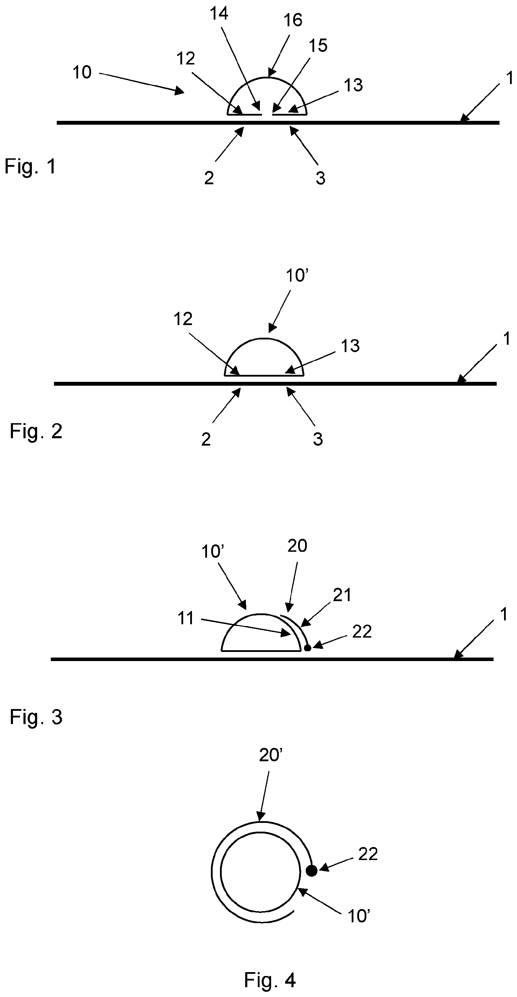

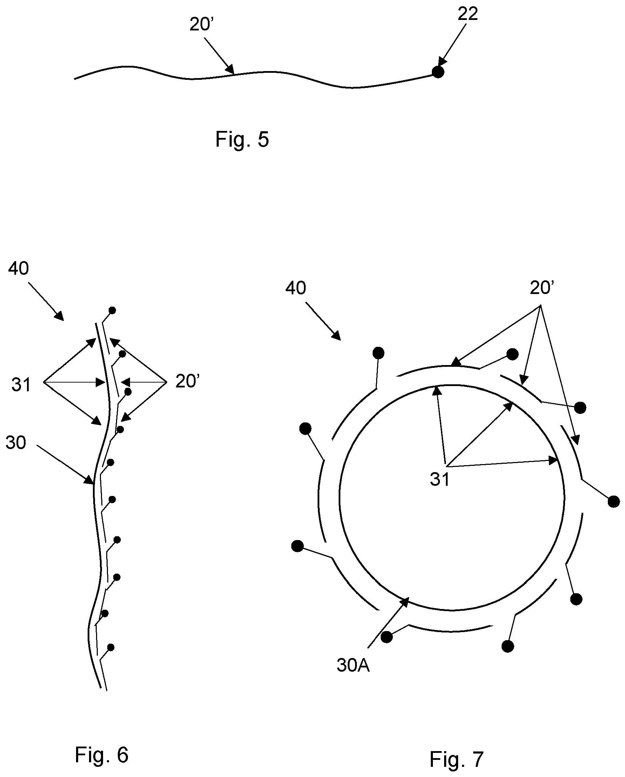

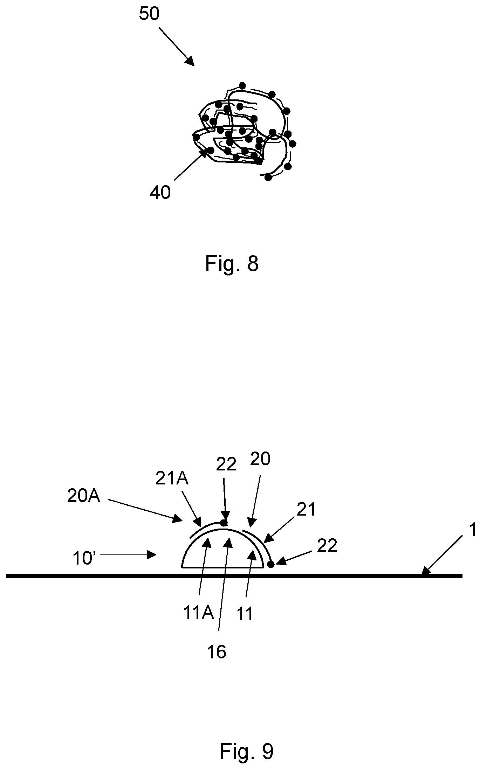

An aspect of the invention thereby relates to a method for detecting at least one target nucleic acid molecule 1 in a sample, see Figs. 1-8 and 18. The method comprises contacting the sample in step S1 with padlock probes 10 comprising at their 5' and 3' ends 14, 15 target-binding regions 12, 13 complementary to probe-binding regions 2, 3 in the at least one target nucleic acid molecule 1, see Fig. 1. A next step S2 comprises joining the 5' and 3' ends 14, 15 of the padlock probes 10 while the target-binding regions 12, 13 are hybridized to the probe-binding regions 2, 3 to form circular padlock probes 10', see Fig. 2. The method also comprises rolling circle amplifying the circular padlock probes 10' in step S3 with labelled amplification primers 20 comprising a detectable label 22 and a probe-binding region 21 complementary to a primer-binding region 11 of the padlock probes 10 to generate labelled rolling circle products 20', see Figs. 3 to 5. The labelled rolling circle products 20' are contacted in step S4 with oligonucleotides 30, 30A, see Figs. 6 and 7, comprising a plurality of binding regions 31 having a nucleic acid sequence corresponding to at least a portion of the padlock probes 10 outside of the primer-binding region 11 to form an agglutinate 50 of labelled complexes 40 between the oligonucleotides 30, 30A and the labelled rolling circle products 20', see Fig. 8. The agglutinate 50 of the labelled complexes 40 are then detected in step S5 to detect the at least one target nucleic acid molecule 1.

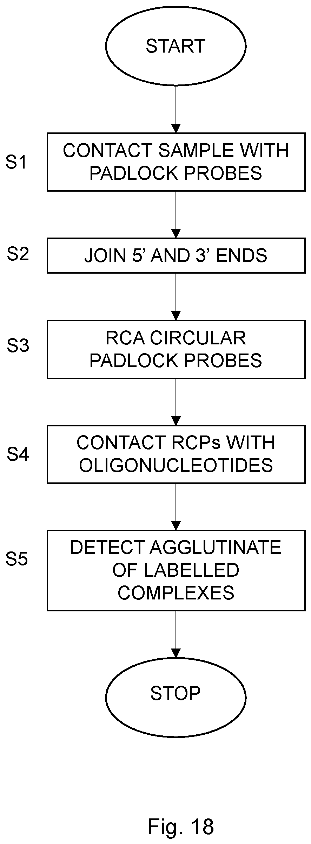

The method as shown in Fig. 18 thereby produces an agglutinate 50 of labelled complexes 40 if the sample contained the at least one target nucleic acid molecule 1. This means that presence of the agglutinate 50 of the labelled complexes 40 in step S5 of Fig. 18 is an indication of presence of the at least one target nucleic acid molecule 1 in the sample 1.

The at least one target nucleic acid molecule 1 detected by the method as shown in Fig. 18 could be any nucleic acid molecule, such as a deoxyribonucleic acid (DNA) molecule, a ribonucleic acid (RNA) molecule, or a nucleic acid molecule comprising synthetic nucleotides or nucleotide analogues, such as locked nucleic acid (LNA), peptide nucleic acid (PNA), etc. The at least one target nucleic acid molecule 1 could be single-stranded or double-stranded.

Illustrative, but non-limiting, examples of target DNA molecules include genomic DNA molecules, cell-free DNA (cfDNA) molecules, circulating tumor DNA (ctDNA) molecules, complementary DNA (cDNA) molecules, or indeed any other DNA molecule from any source, such as viral, bacterial or fungal DNA molecules.

Illustrative, but non-limiting, examples of target RNA molecules include viral, bacterial or fungal RNA molecules, or indeed RNA molecules from any source, such as RNA genomic molecules, complementary RNA (cRNA) molecules, RNA molecules from virions (vRNA), micro RNA (miRNA) molecules, small interfering RNA (siRNA) molecules, messenger RNA (mRNA) molecules, transfer RNA (tRNA) molecules, ribosomal RNA (rRNA) molecules, such as 16S rRNA molecules, small nuclear RNA (snRNA) molecules, small nucleolar RNA (snoRNA) molecules, extracellular RNA (exRNA) molecules, piwi-interacting RNA (piRNA) molecules and long non-coding RNA molecules.

The target nucleic acid molecule is preferably present in a sample, such as a biological sample, for instance, from a subject, such as an animal subject, preferably a mammal subject, and more preferably a human subject. In such a case, the biological sample could be a body fluid sample, such as a blood sample, a blood plasma sample, a blood serum sample, a saliva sample, a sputum sample, a mucosal sample, such as a nasal mucosal lining fluid sample, an oropharyngeal sample, a nasopharyngeal sample, a nasal sample, a bronchoalveolar lavage fluid (BALF) sample, a breast milk sample, a cerebrospinal fluid sample, a urine sample, a feces sample, a tear fluid sample or an endometrial fluid sample, or a body tissue sample, such as a biopsy sample, or a cell sample.

The sample may contain any pathogen or cellular material, including prokaryotic cells, eukaryotic cells, viruses, bacteria, fungi, bacteriophages, mycoplasmas, protoplasts and organelles. For instance, the sample may contain pathogens, such as bacteria, fungi and/or viruses, isolated from a clinical sample taken from a subject. In such a sample, the at least one target nucleic acid molecule may be at least one fungal nucleic acid molecule, at least one bacterial nucleic acid molecule and/or at least one viral nucleic acid molecule.

Other examples include environmental samples including, but not limited to, soil samples, water samples, food samples, etc.

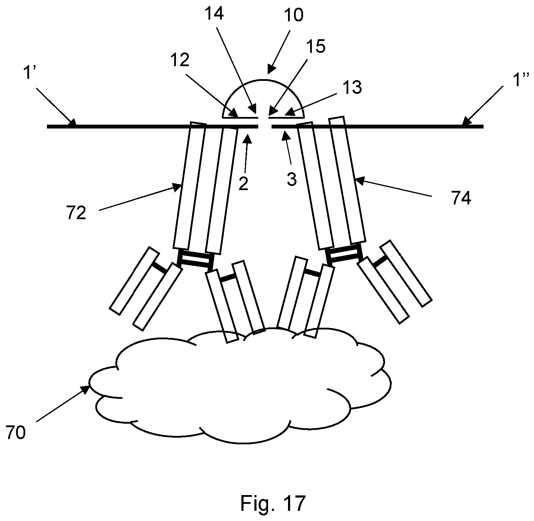

In an embodiment, the at least one target nucleic acid molecule is indicative of the presence of an analyte. For instance, the at least one target nucleic acid molecule could be a reporter for the analyte. Such a reporter can then be used or generated during an assay for an analyte, such as a target molecule, which is further described herein in connection Fig. 17. The target nucleic acid molecule may then act as a reporter and be in the form of a tag or label attached to or forming part of one or more target-binding molecules. A target nucleic acid molecule as a reporter is optionally generated during an assay or in connection with detection of such a target molecule, for example by a ligation reaction in a proximity ligation assay, an extension reaction in a proximity extension assay, or by a cleavage reaction. In such a case, the at last one target nucleic acid molecule could be at least one synthetic or artificial nucleic acid molecule.

The sample may optionally be treated or processed prior to contacting the sample with the padlock probes 10 in step S1 of Fig. 18. For instance, any target nucleic acid molecules 1 could be isolated, separated or removed from an original sample to get an enriched sample that is used in step S1. Other examples of sample processing include fragmenting larger nucleic acid molecules present in the sample into shorter nucleic acid fragments, at least a portion of which are used as target nucleic acid molecules 1.

The method of the invention uses padlock probes 10 that are contacted in step S1 with the sample. The padlock probes 10 of the invention comprise, from their 5' end 14 towards their 3' end 15, a first target-binding region 12, a connecting bridge 16, and a second target-binding region 13. The connecting bridge 16, also referred to as backbone region, connects the two target-binding regions 12, 13 present at the 5' and 3' ends 14, 15 of the padlock probes 10, see Fig. 1. This connecting bridge 16 comprises the primer-binding region 11, to which the labelled amplification primers 20 can bind in step S3.

Padlock probe 10 as used herein refers to any probe capable being circularized following hybridization to a target nucleic acid molecule 1.

The target-binding regions 12, 13 of the padlock probes 10 are complementary to probe-binding regions 2, 3 in the at least one target nucleic acid molecule 1.

Complementary as used herein refers both to complete complementarity of nucleotide sequences, in some cases referred to as an identical sequence, as well as complementarity sufficient to achieve the desired binding of nucleotide sequences. Complementary refers to the standard base pairing rules between G-C, A-T and A-U. Certain nucleotides not commonly found in natural nucleotide sequences or chemically synthesized as mentioned in the foregoing may be included in the nucleotide sequences described herein. Complementarity need not be perfect. In clear contrast, stable duplexes may contain mismatched base pairs, degenerative, or unmatched nucleotides. Complementary is characterized by the capacity for precise pairing of purine and pyrimidine bases of one sequence (strand, oligonucleotide) of a nucleic acid molecule to another sequence of a nucleic acid molecule, such that the order of purine and pyrimidine bases matches and binds to (hybridizes with) the other, complementary sequence.

The complementarity between the target-binding regions 12, 13 and the probe-binding regions 2, 3 allows the target-binding regions 12, 13 of the padlock probes 10 to hybridize to the probe-binding regions 2, 3 of the at least one target nucleic acid molecule 1. Thus, the at least one target nucleic acid molecule 1 is contacted with the padlock probes 10 under hybridization conditions, during which the first target-binding region 12 complementary to a first probe-binding region 2 of the at least one target nucleic acid molecule 1 hybridizes to this first probe-binding region 2 and the second target-binding region 13 complementary to a second probe-binding region 3 of the at least one target nucleic acid molecule 1 hybridizes to this second probe-binding region 3 as shown in Fig. 1. First and second as used herein for the first and second probe-binding region 2, 3 and the first and second target-binding region 12, 13 do not denote any sequence of order, at which these regions 2, 12; 3, 13 hybridize to each other.

Hybridization or hybridization condition denotes the process in which single-stranded nucleotide sequences anneal to complementary nucleotide sequences. Such annealing between complementary nucleotide sequences is dependent on several parameters including, for instance, ionic strength, temperature, length of the target-binding regions 12, 13, and G-C-nucleotides content of the target-binding regions 12, 13.

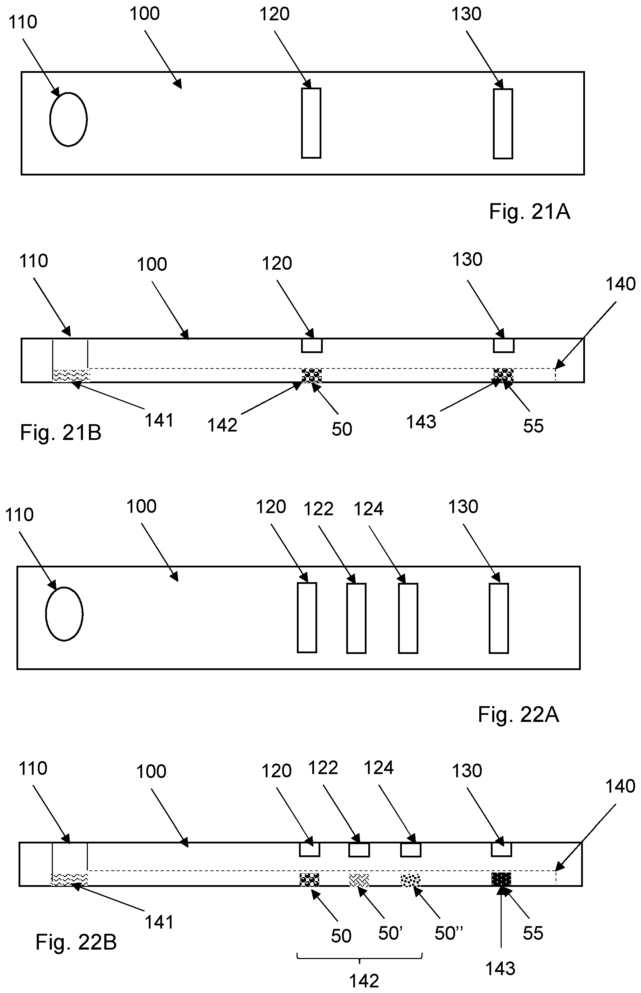

Step S1 in Fig. 18 could be performed according to various embodiments. For instance, the padlock probes 10 could be added to the sample, which is then present in a suitable vessel, such as a well of a multi-well plate, a reaction tube, for instance an Eppendorf^{®} tube, etc. Alternatively, the padlock probes 10 could be pre-loaded in such a well or reaction tube and then the sample is added thereto. It is also possible to use a microfluidic chip 100, see Figs. 21A-22B pre-loaded with, among others, the padlock probes 10 and comprising a sample input or inlet 110, to which the sample is added. A further embodiment is to mix the sample with the padlock probes 10 and then adding the mixture to the well, reaction tube or microfluidic chip 100.

The next step S2 in Fig. 18 comprises joining the 5' and 3' ends 14, 15 of the padlock probes 10 while the target-binding regions 12, 13 are hybridized to the probe-binding regions 2, 3 to form the circular padlock probes 10', see Fig. 2. The joining of the 5' and 3' ends 14, 15 of the padlock probes 10 could be a direct join of the 5' and 3' ends 14, 15 or an indirect join of the 5' and 3' ends 14, 15.

In an embodiment, step S2 in Fig. 18 comprises ligating the 5' and 3' ends 14, 15 of the padlock probes 10 while the target-binding regions 12, 13 are hybridized to the probe-binding regions 2, 3 to form the circular padlock probes 10'. In such an embodiment, a ligating agent is used to ligate the 5' and 3' ends 14, 15 of the padlock probes 10. In an embodiment, the ligating agent is a ligase, and in particular a DNA ligase. A ligase is an enzyme that facilitates the joining of nucleotide strands together by catalyzing the formation of a phosphodiester bond. Any ligase capable of ligating together the 5' and 3' ends 14, 15 of the padlock probes 10 while the target-binding regions 12, 13 are hybridized to the probe-binding regions 2, 3 can be used according to the embodiments.

In a particular embodiment, the ligating agent is a thermostable ligating agent, preferably a thermostable ligase, and in particular a thermostable DNA ligase. For instance, the thermostable ligase could be selected from the illustrative group comprising Ampligase^{®} DNA ligase, Taq DNA ligase, Pfu DNA ligase and 9°N^{™} DNA ligase. Other non-limiting, but illustrative, examples of DNA ligases include Chlorella virus DNA ligase, also referred to as *Paramecium bursaria* Chlorella virus 1 (PBCV-1) DNA ligase or SplintR ligase, *Escherichia coli* DNA ligase encoded by the lig gene; T4 or T7 DNA ligase from bacteriophage T4 or T7; DNA ligase I, II, III or IV. An illustrative, but non-limiting, example of RNA ligases is T4 RNA ligase 2, also referred to as T4 Rnl2 or gp24.1.

Optionally, step S2 of Fig. 18 comprises adding a ligating agent, preferably a ligase, more preferably a DNA ligase, to the sample or a mixture of the sample and the padlock probes 10 to ligate together the 5' and 3' ends 14, 15 of the padlock probes 10. Alternatively, the ligating agent could be pre-mixed with the padlock probes 10.

In an embodiment, the 5' end 14 of the padlock probes 10 comprises a 5' phosphate group to facilitate ligation of the 5' and 3' ends 12, 14 using the ligating agent.

In the above-described embodiments, the padlock probes 10 hybridize to the at least one target nucleic acid molecule 1 with the 5' and 3' ends 14, 15 directly adjacent to each other to facilitate direct ligation of the 5' and 3' end 14, 15.

In other embodiments, the target-binding regions 12, 13 of the padlock probes 10 hybridize to the probe-binding regions 2, 3 of the at least one target nucleic acid molecule 1 with a gap between the 5' and 3' ends 14, 15. The joining of the 5' and 3' ends 14, 15 in step S2 is then an indirect joining of these ends 14, 15. In such a case, the gap between the 5' and 3' ends 14, 15 is filled with at least one gap-filling oligonucleotide or molecular inversion probe that is capable of binding to the target nucleic acid molecule 1 at one or more regions in between the probe-binding regions 2, 3. Alternatively, or in addition, the gap can be filled by extension of the 3' end 15 of the padlock probes 10 while hybridized to the at least one target nucleic acid molecule 1. In such a case, the target nucleic acid molecule 1 acts as a template for the extension with the padlock probe 10 acting as primer. Such an extension is performed by a polymerase and nucleotides (dNTPs) added to the sample or a mixture of the sample and padlock probes 10, or pre-mixed with the padlock probes 10. In the above-described embodiments, a ligating agent, such as the above-described ligases, could join the 5' and 3' ends 14, 15 and the gap-filling oligonucleotide(s) or the 5' end and the extended 3' end 15.

Examples of polymerases that can be used for such an extension include polymerases used in step S3 and further discussed below.

Optionally, the polymerase used for 3' end extension could be inactivated prior to rolling circle amplification in step S3. Such a polymerase inactivation can be performed according to well-known techniques including, but not limited to, heat inactivation.

The formed circular single stranded padlock probes 10' are then amplified in step S3 by so-called RCA using labelled amplification primers 20 to generate the labelled RCPs 20'. RCA uses a strand-displacing polymerase to extend the labelled amplification primers 20 hybridized to the primer-binding region 11 of the circular padlock probes 10', see Figs. 3 and 4. The strand displacing activity of the polymerase displaces the extended labelled amplification primers 20 effectively causing the circular padlock probes 10' to "roll" during RCA.

Illustrative, but non-limiting, examples of strand-displacing polymerases that could be used in step S3 include Phi29 DNA polymerase, Bst polymerase, Klenow fragment, and derivatives thereof.

The strand-displacing polymerase and nucleotides (dNTPs) needed for RCA could be added to sample or the mixture of the sample and the padlock probes 10, or are pre-mixed with the padlock probes 10.

The labelled amplification primers 20 used in the RCA of step S3 in Fig. 18 comprise a probe-binding region 21 complementary to the primer-binding region 11 of the padlock probes 10. Accordingly, the labelled amplification primers 20 are capable of hybridizing to this primer-binding region 11 of the circular padlock probes 10'. The labelled amplification primers 20 could be added together with strand-displacing polymerase to the sample or the mixture of the sample and the padlock probes 10, or could be pre-mixed with the padlock probes 10. Thus, the labelled amplification primers 20 do not significantly interfere with the joining of the 5' and 3' ends 14, 15 of the padlock probes 10 and could thereby be present in the reaction mixture during such a joining in step S2.

The labelled amplification primers 20 comprise a detectable label 22, preferably at or in connection with their 5' ends. The detectable label 22 enables detection of the labelled complexes 40 produced in step S4 if the sample contained the at least one target nucleic acid molecule 1. The label 22 could be any detectable label 22. Illustrative, but non-limiting, examples of such detectable labels 22 include beads, microparticles, nanoparticles, fluorophores, radiolabels, metal-containing labels, colorimetric labels, etc.

In an embodiment, the detectable labels 22 are detectable beads 22. In this embodiment, step S3 comprises RCA the circular padlock probes 10 with the labelled amplification primers 20 comprising a detectable bead 22 and the probe-binding region 21 to generate bead-labelled RCPs 20'.

The detection of the agglutinate 50 of the complexes 40 incorporating the detectable label 22 in step S5 can then be performed according to various embodiments depending on the particular detectable label 22. For instance, agglutinates 50 of complexes 40 incorporating beads, microparticles, nanoparticles, metal-containing labels or colorimetric labels could be detected by visible inspection or light microscopy, whereas fluorescence microscopy could be used to detect agglutinates 50 of complexes 40 comprising fluorophores 22 and autoradiography could be used for detection of agglutinates 50 of radiolabeled complexes 40. Further, colorimetric readout by image analysis of images captured by a camera could be used to detect colorimetric labels.

Step S4 of Fig. 18 comprises contacting the labelled RCPs 20' with oligonucleotides 30, 30A comprising a plurality of binding regions 31 having a nucleic acid sequence corresponding to at least a portion of the padlock probes 10 outside of the primer-binding region 11 to form an agglutinate 50 of labelled complexes 40 between the oligonucleotides 30, 30A and the labelled RCPs 20', see Figs. 6-8. This means that the oligonucleotides 30, 30A comprises a plurality of binding regions 31 each capable of binding to a respective labelled RCP 20'. Accordingly, the oligonucleotides 30, 30A capture the labelled RCPs 20' produced in the RCA in step S3.

The binding regions 31 of the oligonucleotides 30, 30A correspond to at least a portion of the padlock probes 10 outside of the primer-binding region 11. This at least a portion of the padlock probes 10 is preferably a portion of the connecting bridge 16 outside of the primer-binding region 11. The at least a portion of the padlock probes 10 corresponds to the binding regions 31 and thereby enables the binding regions 31 to hybridize to the labelled RCPs 20' at a respective portion of the repeats that is complementary to the at least a portion of the padlock probes 10. This means that this at least a portion of the padlock probes 10 is different than and preferably non-overlapping with the primer-binding region 11. The at least a portion of the padlock probes 10 may, though, at least partly overlap into the primer-binding region 11 with a short number of nucleotides as long as the binding regions 31 preferentially bind to the labelled RCPs 20' over the labelled amplification primers 20. This means that the oligonucleotides 30, 30A bind the labelled RCPs 20' but not the labelled amplification primers 20 or at least has preferential binding of the labelled RCPs 20' over the labelled amplification primers 20.

In an embodiment, the oligonucleotides 30 are linear single-stranded oligonucleotides 30 comprising the plurality of binding regions 31 as shown in Fig. 6. In another embodiment, the oligonucleotides 30A are circular single-stranded oligonucleotides 30A comprising the plurality of binding regions 31 as shown in Fig. 7. It is also possible to use a combination of linear oligonucleotides 30 and circular oligonucleotides 30A in step S4.

In an embodiment, the oligonucleotides 30A are circular oligonucleotides 30A. In such an embodiment, step S4 comprises contacting the labeled RCPs 20' with circular oligonucleotides 30A comprising the plurality of binding regions 31 to form the agglutinate 50 of the labelled complexes 40 between the circular oligonucleotides 30A and the labelled RCPs 20'.

The oligonucleotides 30, 30A with captured labelled RCPs 20' become entangled forming a ball or "blob", i.e., an agglutinate 50 as indicated in Fig. 8. Such a ball or blob is detectable due to the presence of a plurality of labels 22 carried by the labelled RCPs 20' hybridized to the oligonucleotides 30, 30A'. Accordingly, the formed ball or "blob" can be detected as mentioned in the foregoing.

In an embodiment, step S4 in Fig. 18 comprises contacting the labelled RCPs 20' with the oligonucleotides 30, 30A to form an agglutinate 50 of multiple, i.e., at least two, labelled complexes 40, see Fig. 8. In such an embodiment, step S5 comprises detecting the agglutinate 50 of the multiple labelled complexed 40 to detect the at least one target nucleic acid molecule 1. Hence, in this embodiment, multiple labelled complexes 40 are entangled to form an agglutinate 50 comprising multiple such labelled complexes 40. Fig. 24 illustrates such agglutinates 50 comprising colored beads as labels 22.

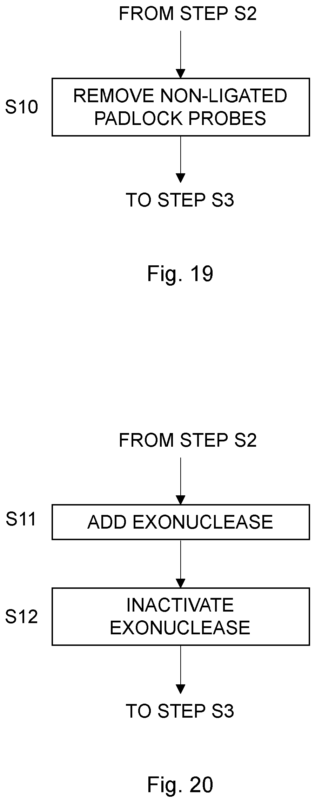

Fig. 19 is a flow chart illustrating an additional, optional step of the method shown in Fig. 18. The method continues from step S2 in Fig. 18 and continues to step S10. This step S10 comprises removing any non-joined padlock probes 10 following joining the 5' and 3' ends 14, 15 of the padlock probes 10 in step S2 but prior to RCA the circular padlock probes 10 in step S3. The method then continues to step S3 in Fig. 18.

Fig. 20 is a flow chart illustrating an embodiment of the removing step S10 in Fig. 19. In this embodiment, the method comprises adding an exonuclease to the sample in step S11 to enzymatically degrade the non-joined padlock probes 10. The method also comprises inactivating the exonuclease in step S11.

The exonuclease added to the sample in step S11 enzymatically cleaves nucleotides one at a time from the 5' end 14 or the 3' end 15 of the non-circularized padlock probes 10. The exonuclease thereby catalyzes a hydrolyzing reaction that breaks phosphodiester bonds at either the 5' end 14 or the 3' end 15. The exonuclease will not degrade the circular padlock probes 10' following joining of the 5' and 3' ends 14, 15 of the padlock probes 10.

Illustrative, but non-limiting, examples of exonucleases that could be used in the embodiment shown in Fig. 20 include exonuclease I, II, III, IV, V, VI, VII, and VIII.

The inactivation of the exonuclease in step S12 can be performed according to well-known techniques, including heat inactivation.

In this embodiment, the labelled amplification primers 20 are preferably added to the sample following inactivation of the exonuclease to prevent the exonuclease from degrading the labelled amplification primers 20. Alternatively, if the labelled amplification primers 20 comprises the detectable label 22 at their 5' ends, then the labelled amplification primers 20 could be present when the exonuclease is added if the exonuclease is a 5' → 3' exonuclease.

In another embodiment, the non-joined padlock 10 could be removed in step S10 by washing. In such an embodiment, a solid phase is used to capture the circular padlock probes 10'. For instance, capturing oligonucleotides that will bind only circular padlock probes 10' could be immobilized on the solid phase, such as by streptavidin-biotin binding. In such a case, the capturing oligonucleotides 30, 31 will not capture free padlock probes 10, i.e., non-circular padlock probes 10. These free padlock probes 10 will be present in the solution and can then be washed away, such as by using a washing buffer. The streptavidin-biotin binding could, following the washing step, be released via competition reaction to thereby release the circular padlock probes 10' and RCA can be performed.

In an embodiment, the padlock probes 10 could be so-called self-destruction padlock probes 10. In such an embodiment, the 3' arm of the padlock probes 10 is designed to enable hybridization to the padlock probe 10 with only a few nucleotides. This means that if the padlock probe 10 is not ligated, the padlock probe 10 will form a hairpin structure once the polymerase is added.

Removal of non-joined padlock probes 10 in step S10 could be beneficial to reduce the risk of non-joined padlock probes 10 binding to the RCPs 20' forming a double-stranded product. Such a binding of non-joined padlock probes 10 to the RCPs 20' could compete with binding of the RCPs 20' to the oligonucleotides 30, 30A. Non-joined padlock probes 10 could also be extended by the polymerase during RCA, and thereby using up a portion of the polymerase, thereby lowering the amount of formed RCPs 20'

In an embodiment, RCA in step S3 comprises RCA the circular padlock probes 10' with the labelled amplification primers 20 to generate the labelled RCPs 20' comprising up to 10 repeats of a nucleic acid sequence complementary to a nucleic acid sequence of the padlock probes 10. Hence, in this embodiment, the labelled RCPs 20' are concatemers comprising up to 10 copies of a nucleic acid sequence complementary to the circular padlock probes 10'. In a preferred embodiment, the labelled RCPs 20' comprise up to 7 repeats, and preferably up to 5 repeats of the nucleic acid sequence complementary to the nucleic acid sequence of the padlock probes 10. In a currently preferred embodiment, the labelled RCPs 20' comprise from 1 up to 10 repeats, preferably from 1 up to 7 repeats, and more preferably from 1 up to 5 repeats of the nucleic acid sequence complementary to the nucleic acid sequence of the padlock probes 10.

Hence, the labelled RCPs 20' generated according to these embodiments require comparatively short RCA times to thereby produce labelled RCPs 20' with merely one or a few repeats. The present invention still enables detection of the labelled RCPs 20' even though they merely contain such few repeats by using the oligonucleotides 30, 30A that capture multiple such labelled RCPs 20' in step S4. Hence, the present invention enables visual detection of the labelled RCPs 20' with merely 10 min of RCA (Fig. 24), whereas visual detection of the labelled RCPs 20' without usage of the oligonucleotides 30, 30A of the invention require 40 min of RCA to generate a high number of repeats of the nucleic acid sequence complementary to the nucleic acid sequence of the padlock probes 10, and thereby sufficiently long labelled RCPs 20'.

In an embodiment, step S4 of Fig. 18 comprises contacting the labelled RCPs 20' with the oligonucleotides 30, 30A comprising at least 100 repeats of the binding region 31. In a preferred embodiment, the oligonucleotides 30, 30A comprise at least 500 repeats of the binding region 31, preferably from 500 up to 1500 repeats of the binding region 31.

Hence, the oligonucleotides 30, 30A comprise a comparatively high number of such binding regions 31 to thereby enable binding of a high number of labelled RCPs 20' per such an oligonucleotide 30, 30A. This means that each oligonucleotide 30, 30A will typically bind a plurality of labelled RCPs 20' and thereby comprise a plurality of detectable labels 22, which facilitates detection of the labelled complexes 40 between the oligonucleotides 30, 30A and the labelled RCPs 20' even though the labelled RCPs 20' merely comprise few repeats of the nucleic acid sequence complementary to the nucleic acid sequence of the padlock probes 10.

The oligonucleotides 30, 30A of the invention could be regarded as being agglutinate-promoting oligonucleotides 30, 30A as they promote formation of detectable labelled complexes 40 and agglutinates 50 comprising the labelled RCPs 20'. Hence, the oligonucleotides 30, 30A compensate for the reduced length of the labelled RCPs 20' by binding to multiple labelled RCPs 20' that together cause formation of detectable complexes 40 and agglutinates 50 even though the labelled RCPs 20' as such are too small or short to form any detectable agglutinates 50 without the oligonucleotides 30, 30A.

In an embodiment, multiple sets of labelled amplification primers 20, 20A that are complementary to different primer binding regions 11, 11A in the padlock probes 10. This is schematically shown in Fig. 9. In such an embodiment, step S3 of Fig. 18 preferably comprises RCA the circular padlock probes 10' with multiple sets of labelled amplification primers 20, 20A to generate the labelled RCPs 20'. The labelled amplification primers 20, 20A in each set of the multiple sets comprise the same probe-binding region 21, 21A. The probe-binding region 21 of the labelled amplification primers 20 in one set of the multiple sets is complementary to another primer-binding region 11 of the padlock probes 10 than the primer-binding region 21A of the labelled amplification primers 20A in another set of the multiple sets. The labelled amplification primers 20, 20A in the multiple sets have or comprise the same detectable label 22.

In Fig. 9, two sets of different amplification primers 20, 20A are used. In such a case, the amplification primers 20 in the first set comprise a first probe-binding region 21 complementary to a first primer-binding region 11 of the padlock probes 10 and a detectable label 22. Correspondingly, the amplification primers 20A in the second set comprise a second probe-binding region 21A complementary to a second primer-binding region 11A of the padlock probes 10 and the detectable label 22. This means that the connecting bridge 16 comprises, in this embodiment, two primer-binding regions 11, 11A.

The embodiments are not limited to using merely two sets of labelled amplification primers 20, 20A but could include more than two different sets.

In some embodiments, it could be beneficial to use multiple sets of labelled amplification primers 20, 20A to increase the number of labelled RCPs 20' produced in the RCA of step S3. As a consequence, a high number of labelled RCPs 20' can be produced even though the RCA of step S3 is run for a short period of time. This in turn means that a high number of labelled RCPs 20' are available to bind to the oligonucleotides 30, 30A in step S4, which in turn facilitates formation of detectable labelled complexes 40. This is possible since the labelled amplification primers 20, 20A in the multiple sets comprise the same type of detectable label 22, thereby producing the same detectable signal.

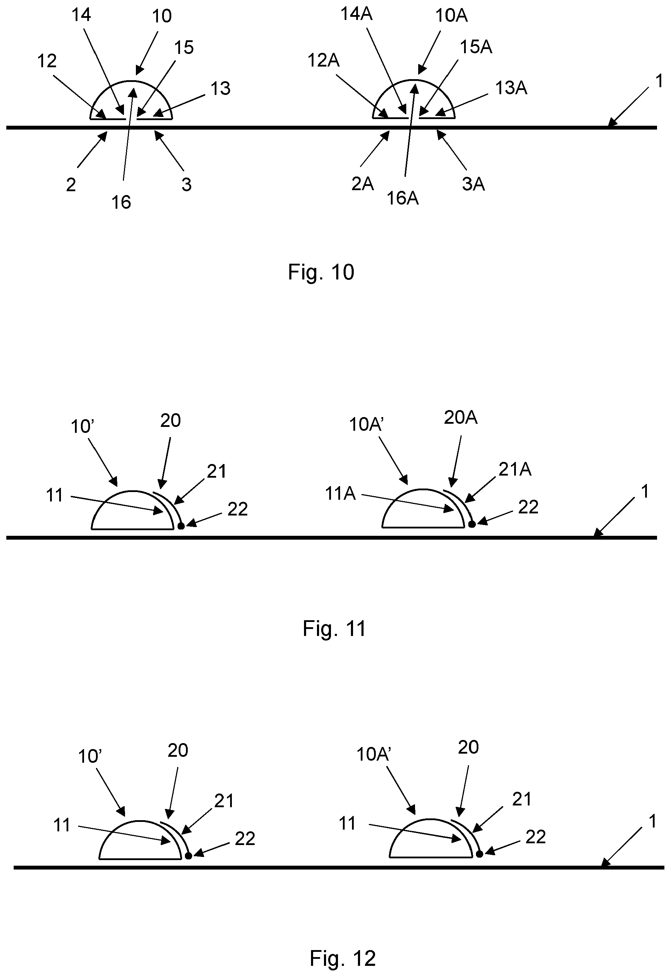

Another embodiment of increasing the detectable signal is to use multiple sets of padlock probes 10, 10A as shown in Figs. 10 and 11. In such an embodiment, step S1 in Fig. 18 comprises contacting the sample with multiple sets of padlock probes 10, 10A. The padlock probes 10, 10A in each set of the multiple sets comprise the same target-binding regions 12, 13; 12A, 13A. The target-binding regions 12, 13 of the padlock probes 10 in one set of the multiple sets are complementary to other probe-binding regions 2, 3 in the at least one target nucleic acid molecule 1 than the target-binding regions 12A, 13A of the padlock probes 10A in another set of the multiple sets. Step S3 comprises, in this embodiment, RCA the circular padlock probes 10', 10A' with multiple sets of labelled amplification primers 20, 20A to generate multiple sets of labelled rolling circle products 20'. The labelled amplification primers 20, 20A in each set of the multiple sets comprise the same probe-binding region 21, 21A. The probe-binding region 21 of the labelled amplification primers 20 in one set of the multiple sets is complementary to another primer-binding region 11 of the padlock probes 10, 10A than the primer-binding region 21A of the labelled amplification primers 20A in another set of the multiple sets. The labelled amplification primers 20, 20A in the multiple sets have or comprise the same detectable label 22.

In Fig. 10, two sets of different padlock probes 10, 10A are used. In such a case, the padlock probes 10 in a first set comprises a first set of target-binding regions 12, 13 that are complementary to a first set of probe-binding regions 2, 3 in the at least one target nucleic acid molecule 1. Correspondingly, the padlock probes 10A in a second set comprises a second set of target binding regions 12A, 13A that are complementary to a second set of probe-binding regions 2A, 3A in the at least one target nucleic acid molecule 1. This means that padlock probes 10, 10A in the different sets hybridize to different regions 2, 3; 2A, 3A in the at least one target nucleic acid molecule 1, see Fig. 10. Furthermore, the labelled amplification primers 20 in a first set comprise a first probe-binding region 21 complementary to a first primer-binding region 11 of the padlock probes 10 in the first set and the detectable label 22. Correspondingly, the labelled amplification primers 20A in a second set comprise a second probe-binding region 21A complementary to a second primer-binding region 11A of the padlock probes 10A in the second set and the detectable label 22.

In this embodiment, two species or sets of labelled RCPs 20' will be produced in the RCA of step S3. The two species of labelled RCPs 20' comprise different nucleic acid sequences since they are produced by RCA of different padlock probes 10, 10A having at least partly different nucleic acid sequences 2, 3, 11; 2A, 3A, 11A.

In an embodiment, the padlock probes 10, 10A in the multiple sets comprise a respective portion having the same nucleic acid sequence as the nucleic acid sequence of the binding regions 31 of the oligonucleotides 30, 30A. This same portion corresponds to at least a portion of the connecting bridge 16, 16A outside of the primer-binding region 11, 11A. In such an embodiment, the oligonucleotides 30, 30A can capture the different species or sets of labelled RCPs 20' since at least a portion of the nucleic acid sequence of labelled RCPs 20' is the same for the different sets of padlock probes 10, 10A and therefore for the labelled RCPs 20'. Hence, a single species of oligonucleotides 30, 30A could thereby be used to capture the multiple sets of labelled RCPs 20'.

In another embodiment, step S4 of Fig. 18 comprises contacting the multiple sets of labelled RCPs 20' with multiple sets of oligonucleotides 30, 30A to form an agglutinate 50 of multiple sets of labelled complexes 40 between the oligonucleotides 30, 30A and the labelled RCPs 20'. In this embodiment, the oligonucleotides 30, 30A in each set of the multiple sets comprise the same binding regions 31. The binding region 31 of the oligonucleotides 30, 30A in one set of the multiple sets has a nucleic acid sequence different than the nucleic acid sequence of the binding region 31 of the oligonucleotides in another set of the multiple sets. In this embodiment, different species of oligonucleotides 30, 30A are used to capture the different species of labelled RCPs 20'. This means that multiple sets of labelled complexes 40 are formed but all these labelled complexes 40 comprise the same type of detectable label 22 and thereby produce the same detectable signal in the form of a detectable agglutinate 50.

In a further embodiment, the labelled amplification primers 21, 21A of the two sets have different detectable labels 22. In such a case, a target nucleic acid molecule 1 comprising the first and second sets of probe-binding regions 2, 3; 2A, 3A will produce two detectable signals in the form of differently labelled agglutinates 50 or an agglutinate 50 comprising labelled complexes 40 with different labels 22. Hence, in such a case a correct detection of the target nucleic acid molecule 1 in the sample would imply detection of two different labelled complexes 40. In such an embodiment, step S1 in Fig. 18 comprises contacting the sample with multiple sets of padlock probes 10, 10A. The padlock probes 10, 10A in each set of the multiple sets comprise the same target-binding regions 12, 13; 12A, 13A. The target-binding regions 12, 13 of the padlock probes 10 in one set of the multiple sets are complementary to other probe-binding regions 2, 3 in the at least one target nucleic acid molecule 1 than the target-binding regions 12A, 13A of the padlock probes 10A in another set of the multiple sets. Step S3 comprises, in this embodiment, RCA the circular padlock probes 10', 10A' with multiple sets of labelled amplification primers 20, 20A to generate multiple sets of labelled rolling circle products 20'. The labelled amplification primers 20, 20A in each set of the multiple sets comprise the same probe-binding region 21, 21A and the same detectable label 22. The probe-binding region 21 of the labelled amplification primers 20 in one set of the multiple sets is complementary to another primer-binding region 11 of the padlock probes 10, 10A than the primer-binding region 21A of the labelled amplification primers 20A in another set of the multiple sets. Furthermore, the detectable label 22 of the labelled amplification primers 20 in one set of the multiple sets is different than the detectable label of the labelled amplification primers 20A in another set of the multiple sets. Step S4 of Fig. 18 comprises contacting the multiple sets of labelled RCPs 20' with multiple sets of oligonucleotides 30, 30A to form at least one agglutinate 50 of multiple sets of labelled complexes 40 between the oligonucleotides 30, 30A and the labelled RCPs 20'. In this embodiment, the oligonucleotides 30, 30A in each set of the multiple sets comprise the same binding regions 31. The binding region 31 of the oligonucleotides 30, 30A in one set of the multiple sets has a nucleic acid sequence different than the nucleic acid sequence of the binding region 31 of the oligonucleotides in another set of the multiple sets. In this embodiment, different species of oligonucleotides 30, 30A are used to capture the different species of labelled RCPs 20'. This means that an agglutinate 50 of multiple sets of labelled complexes 40 or multiple agglutinates 50, each of a respective set of labelled complexes 40, are formed and produce the different detectable signals.

This embodiment could increase the specificity of the method by requiring multiple different detectable signals to confirm presence of the at least one target nucleic acid molecule 1 in the sample.

A further embodiment is shown by Figs. 10 and 12. In this embodiment, step S1 of Fig. 18 comprises contacting the sample with multiple sets of padlock probes 10, 10A. The padlock probes 10, 10A in each set of the multiple sets comprise the same target-binding regions 12, 13; 12A, 13A. The target-binding regions 12, 13 of the padlock probes 10 in one set of the multiple sets are complementary to other probe-binding regions 2, 3 in the at least one target nucleic acid molecule 1 than the target-binding regions 12A, 13A of the padlock probes 10A in another set of the multiple sets, see Fig. 10. However, in this embodiment, the padlock probes 10, 10A in the multiple sets comprise the same primer binding region 11.

This means that only a single set of labelled amplification primers 11 are needed in the RCA of step S3 since the labelled amplification primers 11 can bind to the circular padlock probes 10', 10A' even though these circular padlock probes 10', 10A' partly comprise different nucleic acid sequences 12, 13; 12A, 13A since the primer-binding region 11 is the same in the multiple sets of padlock probes 10, 10A, see Fig. 12.

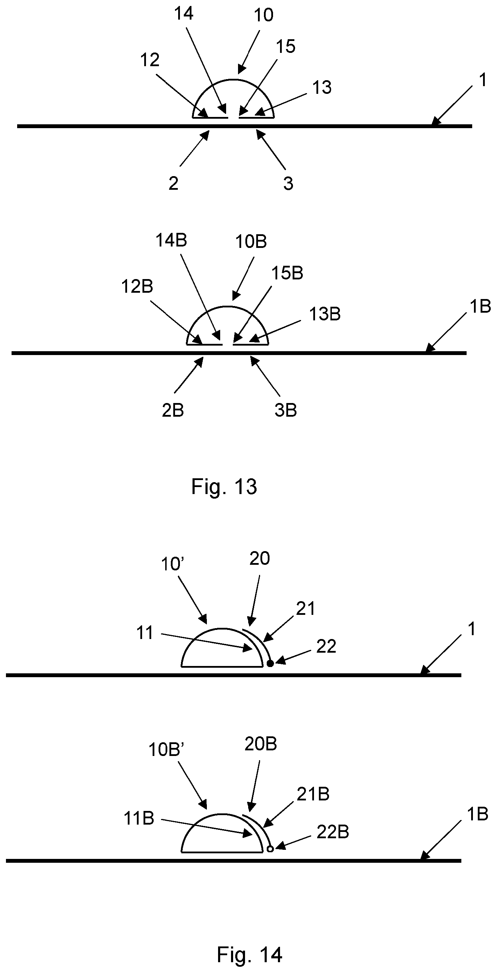

The present invention can be used to detect the presence of multiple different target nucleic acid molecules 1, 1B in a single sample, see Figs. 13 to 16. In such an embodiment, step S1 of Fig. 18 comprises contacting a sample comprising multiple different target nucleic acid molecules 1, 1B with multiple sets of padlock probes 10, 10B, see Fig. 13. The padlock probes 10, 10B in each set of the multiple sets comprise the same target-binding regions 12, 13; 12B, 13B. The target-binding regions 12, 13 of the padlock probes 10 in one set of the multiple sets are complementary to other probe-binding regions 2, 3 in the multiple different target nucleic acid molecules 1, 1B than the target-binding regions 12B, 13B of the padlock probes 10B in another set of the multiple sets.

Step S2 comprises, in this embodiment, joining the 5' and 3' ends 14, 15 of the padlock probes 10, 10B while the target-binding regions 12, 13; 12B, 13B are hybridized to the probe-binding regions 2, 3; 2B, 3B to form circular padlock probes 10', 10B'

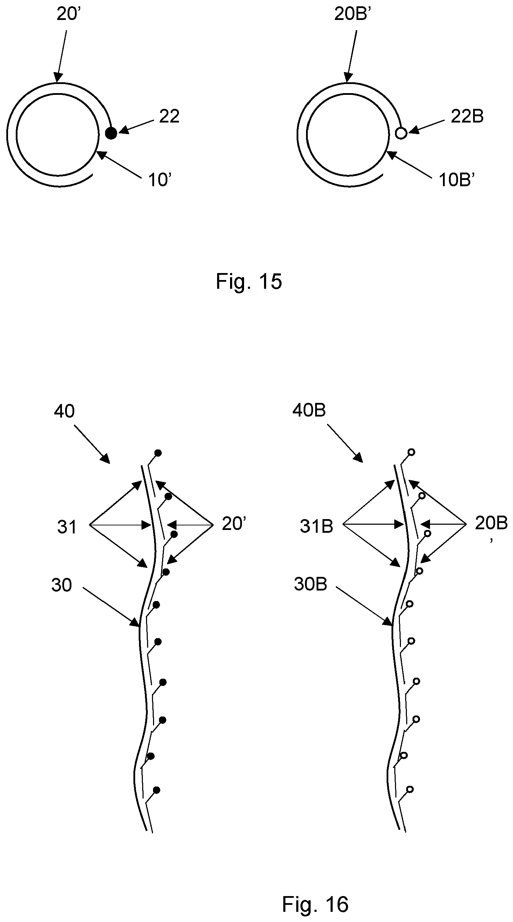

Step S3 comprises, in this embodiment, RCA the circular padlock probes 10', 10B' with multiple sets of labelled amplification primers 20, 20B to generate the labelled RCPs 20', 20B', see Figs. 14 and 15. The labelled amplification primers 20, 20B in each set of the multiple sets comprise the same probe-binding region 21, 21B and the same detectable label 22, 22B. The probe-binding region 21 of the labelled amplification primers 20 in one set of the multiple sets is complementary to another primer-binding region 11 of the padlock probes 10, 10B than the primer-binding region 21B of the labelled amplification primers 20B in another set of the multiple sets. The detectable label 22 of the labelled amplification primers 20 in one set of the multiple sets is different than the detectable label 22B of the labelled amplification primers 20B in another set of the multiple sets.

The method also comprises, in this embodiment, contacting the labelled RCPs 20', 20B' with multiple sets of oligonucleotides 30, 30B in step S4 to form at least one agglutinate 50 of the labelled complexes 40, 40B between the oligonucleotides 30, 30B and the labelled RCPs 20', 20B', see Fig. 16. In this embodiment, the oligonucleotides 30, 30B in each set of the multiple sets comprise the same binding regions 31, 31B. The binding regions 31 of the oligonucleotides 30 in one set of the multiple sets have different nucleic acid sequences than the binding regions 31B of the oligonucleotides 30B in another set of the multiple sets.

Step S5 comprises detecting at least one agglutinates 50 of multiple labelled complexes 40, 40B with different labels 22, 22B to detect the multiple target nucleic acid molecules 1, 1B.

This step S5 could, thus, involve detecting one agglutinate 50that is a mixture of the multiple labelled complexes 40, 40B with different labels 22, 22B or detecting multiple agglutinates 50, each being an agglutinate 50 of multiple complexed 40, 40B with a respective label 22, 22B.

The above-described and in Figs. 13-16 shown embodiment thereby enables detection of the presence of multiple different target nucleic acid molecules 1, 1B in the same sample. As an example, such an embodiment could be used to detect the presence of multiple pathogens, such as bacteria and/or viruses, in a sample if the padlock probes 10, 10B of the different sets are designed to bind to target nucleic acid molecules 1, 1B from different bacteria and/or viruses.

This embodiment thereby provides a multiplex detection allowing identification of one or more different species of target nucleic acid molecules in a single sample, which thereby could indicate the presence of one or more pathogens.

The embodiments described in the foregoing in connection with Figs. 10-12 can be applied to the embodiment discussed above and in connection with Figs. 13-16. In such a case, multiple sets of padlock probes could be targeting one nucleic acid molecule 1 and one or multiple sets of padlock probes could be targeting another nucleic acid molecule 1B in the same sample.

As mentioned in the foregoing, the embodiments described in connection with Figs. 10-16 are not limited to using two sets of padlock probes 10, 10A, 10B implying that more than two sets could be used.

In an embodiment, the at least one target nucleic acid molecule is not originally present in the sample to be tested. Rather the sample comprises at least one target molecule 70, see Fig. 17. Such a target molecule 70 could then be a molecule other than a nucleic acid molecule or be a complex of one or more nucleic acid molecules and one or more other molecules. As an illustrative example, the target molecule 70 could be a protein, a polypeptide or at least comprise at least one protein or polypeptide domain.

In such a case, target-binding molecules 72, 74 having affinity for, and preferably binding specifically to, the target molecule 70 and comprising a respective target nucleic acid molecule 1', 1" could be used. This means that the target nucleic acid molecules 1', 1" are thereby introduced by the target-binding molecules 72, 74. In such a case, the target-binding molecules 72, 74 bind to different regions, such as epitopes, of the target molecule 70 to bring the target nucleic acid molecules 1', 1" attached to, or forming part of, the target-binding molecules 72, 74 in close proximity of each other allowing a padlock probe 10 to bind to respective probe-binding regions 2, 3 in the respective target nucleic acid molecules 1', 1" as shown in Fig. 17.

The detection of the target nucleic acid molecules 1', 1" in the sample is then indicative of the presence of the target molecule 70 in the sample.

In an embodiment, the target-binding molecules 72, 74 are antibodies, or antigen-binding fragments thereof.

The antibodies 72, 74, or the antigen-binding fragments thereof, specifically bind to the target molecule 70, and preferably to different epitopes on the target molecule 70.

The specificity of an antibody, or an antigen-binding fragment thereof, can be determined based on affinity and/or avidity. The affinity, represented by the equilibrium constant for the dissociation (K_{d}) of an antigen with the antibody, or the antigen-binding fragment thereof, is a measure for the binding strength between an antigenic determinant, i.e., epitope, and an antigen-binding site on the antibody, or the antigen-binding fragment thereof. The lesser the value of K_{d}, the stronger the binding strength between the antigenic determinant and the antibody, or the antigen-binding fragment thereof. Alternatively, the affinity can also be expressed as the affinity constant (Kₐ), which is 1/K_{d}. As will be clear to the skilled person, affinity can be determined in a manner known per se, depending on the specific antigen of interest.

Avidity is the measure of the strength of binding between an antibody, or an antigen-binding fragment thereof, and the pertinent antigen. Avidity is related to both the affinity between an antigenic determinant and its antigen binding site on the antibody, or the antigen-binding fragment thereof, and the number of pertinent binding sites present on the antibody, or the antigen-binding fragment thereof.

Typically, antibodies, or antigen-binding fragments thereof, will bind to their antigen with a dissociation constant (K_{d}) of 10⁻⁵ to 10⁻¹² moles/liter (M) or less, and preferably 10⁻⁷ to 10⁻¹² M or less and more preferably 10⁻⁸ to 10⁻¹² M, i.e., with an association constant (Kₐ) of 10⁵ to 10¹² M⁻¹ or more, and preferably 10⁷ to 10¹² M⁻¹ or more and more preferably 10⁸ to 10¹² M⁻¹.

Typically, any K_{d} value greater than 10⁻⁴ M (or any Kₐ value lower than 10⁴ M⁻¹) is generally considered to indicate non-specific binding.

Specific binding of an antibody, or an antigen-binding fragment thereof, to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art.

In an embodiment, the antibodies 72, 74 are monoclonal antibodies. In other embodiments, the antibodies 72, 74 are polyclonal antibodies, or a combination of monoclonal and polyclonal antibodies.

An antigen-binding fragment of an antibody as used herein can be selected from a group consisting of a single chain antibody, a Fv fragment, a scFv fragment, a Fab fragment, a F(ab')₂ fragment, a Fab' fragment, a Fd fragment, a single-domain antibody (sdAb), a scFv-Fc fragment, and a di-scFv fragment.

In such embodiment, step S1 of Fig. 18 comprises contacting the sample with multiple target-binding molecules 72, 74 binding specifically to a target molecule 70, and padlock probes 10. Each target-binding molecule 72, 74 of the multiple target-binding molecules 72, 74 comprise a respective target nucleic acid molecule 1', 1". The padlock probes 10 comprise at their 5' and 3' ends target-binding regions 12, 13 complementary to probe-binding regions 2, 3 in the respective target nucleic acid molecules 1', 1". Step S2 comprises, in this embodiment, joining the 5' and 3' ends 14, 15 of the padlock probes 10 while the target-binding regions 12, 13 are hybridized to the probe-binding regions 2, 3 and the multiple target-binding molecules 72, 74 are binding to the target molecule 70 to form circular padlock probes 10'. In this embodiment, step S5 comprises detecting the agglutinate 50 of the labelled complexes 40 to detect the respective target nucleic acid molecules 1', 1" and thereby the target molecule 70.

In an embodiment, steps S1-S4 of Fig. 18 are performed in a reaction tube 60, such as an Eppendorf^{®} tube, see Fig. 24, or in a well of a multi-well plate. The detection step S5 can also be performed with the labelled complexes 40 present in the reaction tube 60 or well.

In an embodiment, the above-described steps S1-S4 and optionally also S5 are performed in solution, i.e., without the need for any solid phase.

In another embodiment, the method of the invention is performed using a microfluidic chip 100, which will be further described below in connection with Figs. 21A-22B.

Another aspect of the invention relates to a kit for detecting at least one target nucleic acid molecule 1 in a sample. The kit comprises padlock probes 10 comprising at their 5' and 3' ends 14, 15 target-binding regions 12, 13 complementary to probe-binding regions 2, 3 in at least one target nucleic acid molecule 1. The kit also comprises labelled amplification primers 20 comprising a detectable label 22 and a probe-binding region 21 complementary to a primer-binding region 11 of the padlock probes 10. The kit further comprises oligonucleotides 30, 30A comprising a plurality of binding regions 31 having a nucleic acid sequence corresponding to at least a portion of the padlock probes 10 outside of the primer-binding region 11.

In an embodiment, the kit further comprises a ligase adapted to ligate the 5' and 3' ends 14, 15 of the padlock probes 10 while the target-binding regions 12, 13 are hybridized to the probe-binding regions 2, 3 to form circular padlock probes 10'.

In an embodiment, the kit also comprises a polymerase adapted to rolling circle amplify the circular padlock probes 10' with the labelled amplification primers 20 to generate labelled RCPs 20'.

Examples of ligases and polymerases that could be included in the kit are presented in the foregoing.

The kit may optionally also comprise other components required by the ligase and/or the polymerase, such as nucleotides (dNTPs), buffer, etc.

The kit may also comprise a reaction vessel, such as a reaction tube 60, well, or a microfluidic chip 100, in which to perform the detection of at least one target nucleic acid molecule 1.

The kit of the invention is adapted to perform the method for detecting at least one target nucleic acid molecule 1 as discussed above in connection with Figs. 1-20.

The various embodiments of the padlock probes 10, labelled amplification primers 20, and oligonucleotides 30, 30A discussed in the foregoing in connection with Figs. 1-20 also apply to the kit of the invention.

The invention also relates to a microfluidic chip or device 100, see Figs. 21A-22B. The microfluidic chip 100 comprises a sample input or inlet 110 configured to receive a sample comprising at least one target nucleic acid molecule 1.

The microfluidic chip 100 also comprises a microfluidic channel 140 in fluid connection with the sample input 110. The microfluidic channel 140 comprises a reagent section 141 comprising padlock probes 10 comprising at their 5' and 3' ends 14, 15 target-binding regions 12, 13 complementary to probe-binding regions 2, 3 in the at least one target nucleic acid molecule 1. The reagent section 141 also comprises a ligase adapted to ligate the 5' and 3' ends 14, 15 of the padlock probes 10 while the target-binding regions 12, 13 are hybridized to the probe-binding regions 2, 3 to form circular padlock probes 10". The reagent section 141 further comprises labelled amplification primers 20 comprising a detectable label 22 and a probe-binding region 21 complementary to a primer-binding region 11 of the padlock probes 10. The reagent section 141 additionally comprises a polymerase adapted to rolling circle amplify the circular padlock probes 10' with the labelled amplification primers 20 to generate labelled RCPs 20'.

The microfluidic channel 140 also comprises a detection section 142 comprising oligonucleotides 30, 30A comprising a plurality of binding regions 31 having a nucleic acid sequence corresponding to at least a portion of the padlock probes 10 outside of the primer-binding region 11.

The microfluidic chip 100 also comprises a detection window 120, 122, 124 aligned with at least a portion of the detection section 142 and arranged to enable visual access to the at least a portion of the detection section 142.

The microfluidic chip 100 is thereby a device pre-loaded with all or at least a majority of the reagents and components needed to enable detection of a target nucleic acid molecule 1 in a sample input into the sample input 110. In other words, the reagents and components are preferably pre-loaded to be present in the reagent section 141 but with the oligonucleotides 30, 30A preferably present at the detection section 142. In such case, the reagents and components could all be provided as a mixture in the same part of the microfluidic channel 140. Alternatively, the reagents and components could be distributed at different portions of the microfluidic channel 140. In such a case, the reagent section 141 typically extends along a comparatively longer part of the microfluidic channel 140 as compared to providing all the reagents and components at the same part of the microfluidic channel 140. For instance, the reagents and components could be distributed in the following order, from an upstream position in connection with the sample input 110 in a downstream direction towards the detection section 142, the padlock probes 10, the ligase, the labelled amplification primers 20 and the polymerase, or the padlock probes 10, the labelled amplification primers 20, the ligase and the polymerase as illustrative, but non-limiting, examples.

For instance, the reagent section 141 could comprise an upstream or ligase reagent section comprising the padlock probes 10 and the ligase and a downstream or RCA reagent section comprising the labelled amplification primers 20 and the polymerase.

The sample added to the sample input 110 is a liquid sample potentially comprising the at least one target nucleic acid molecule 1. The liquid part of the sample will then carry the target nucleic acid molecule 1 up to the reagents and components in the reagent section 141 and further carry the resulting circular padlock probes 10', and the labelled RCPs 20' towards the end of the microfluidic channel 140 and thereby past the detection section 142. The joining reaction catalyzed by the ligase and the RCA catalyzed by the polymerase will preferably thereby take place as the liquid is transported along the microfluidic channel 140. The microfluidic channel 140 is then preferably designed to allow sufficient time for the joining and RCA reactions to take place so that a sufficient number of labelled RCPs 20' are produced when reaching the detection section 142. As an example, the microfluidic channel 140 could comprise a matrix or a membrane, such as a nitrocellulose membrane, through which the liquid is migrating. Optionally, wick could be arranged in connection with the end of the microfluidic channel 140 to draw the liquid from the sample input 110 towards the end of the microfluidic channel 140.

The oligonucleotides 30, 30B are preferably provided in the portion of the microfluidic channel 140 aligned with the detection window 120, 122, 124. For instance, the oligonucleotides 30, 30B could be immobilized, such as attached, directly or indirectly, to the wall(s) of the microfluidic channel 140 or to the matrix or membrane if present in the microfluid channel 140. As a consequence, any labelled RCPs 20' transported along the microfluidic channel 140 will be captured by the, preferably immobilized, oligonucleotides 30, 30B to form labelled complexes 40 at the detection section 142.

Such labelled complexes 40 or agglutinates 50 of multiple labelled complexes 40 could then be detected through the detection window 120, 122, 124. In such a case, the detectable label 22 of the labelled amplification primers 20 is preferably visibly detectable, such as in the form of colored beads or microparticles, or a colorimetric label. The presence of the labelled complexes 40 or agglutinates 50 in the detection section 142 could then be verified through the detection window 120, 122, 124. If such labelled complexes 40 or agglutinates 50 become visible through the detection window 120, 122, 124 the sample contained the at least one target nucleic acid molecule 1.

Figs. 21A and 21B illustrate a microfluidic chip 100 with a single detection window 120. Such a microfluidic chip 10 could then be used to detect the presence of a single target nucleic acid molecule 1 in the sample. Figs. 22A and 22B illustrate a microfluidic chip 100 with multiple detection windows 120, 122, 124. In such a case, the detection section 142 comprises multiple portions with oligonucleotides 30, 30B designed to capture different species of labelled RCPs 20', 20B' as discussed above in connection with Figs. 13-16, to thereby produce different labelled complexes or agglutinates 50, 50', 50". In such a case, each detection window 120, 122, 124 is aligned with a respective such portion of the detection section 142 with oligonucleotides 30, 30B. Such a microfluidic chip 100 could then be used to detect presence of multiple different target nucleic acid molecules 1, 1B in a single sample.

In an embodiment, the microfluidic channel 140 of the microfluidic chip 100 also comprises a control section 143 comprising oligonucleotides comprising a plurality of binding regions having a nucleic acid sequence complementary to the probe-binding region 21 of the labelled amplification primers 20. The microfluidic chip 100 comprises, in this embodiment, a control window 130 aligned with the control section 143 and arranged to enable visual access to the control section 143. In such a case, the oligonucleotides present, preferably immobilized, at the control section 143 can capture any labelled amplification primers 20 to form a labelled complex or agglutinate 55 between the oligonucleotides and the labelled amplification primers 20. Such a labelled complex or agglutinate 55 visible through the control window 130 verifies sufficient transport of the liquid with reagents and components from the sample input 110 and the reagent section 141 and up to the control section 143. This control section 143 is preferably arranged downstream of the detection section 142, i.e., with the detection section 142 present between the reagent section 141 and the control section 143.

A labelled complex or agglutinate 55 visible through the control window 130 but no labelled complex or agglutinate 50 visible through any of the detection window(s) 120, 122, 124 indicate a negative result, i.e., the sample does not contain the at least one target nucleic acid molecule 1. However, labelled complexes or agglutinates 50, 50', 50", 55 visible both through the control 130 and through at least one detection window 120, 122, 124 indicate a positive result, i.e., the sample contained the at least one target nucleic acid molecule 1.

The various embodiments of the padlock probes 10, labelled amplification primers 20, and oligonucleotides 30, 30A discussed in the foregoing in connection with Figs. 1-20 also apply to the microfluidic chip 100 of the invention.

### EXAMPLES

This example investigated the feasibility of the present invention showing the production of a visible signal indicating presence of a target nucleic acid molecule in a sample already following 10 minutes of rolling circle amplification, whereas traditional padlock probe and RCA based detection required 40 minutes of rolling circle amplification to produce a visible signal.

10 nM padlock probes with 5' phosphate group (SEQ ID NO: 1-3, Table 1) were mixed with 1U/µl SplintR ligase (New England Biolabs) and 1X SplintR ligation buffer (New England Biolabs) and mixed with 5 nM synthetic target nucleic acid molecules (SEQ ID NO: 4-6, Table 1). The hybridization and ligation were allowed to proceed for 5 min at room temperature (20-25°C). Any unbound padlock probes were removed by exonuclease treatment by adding 1U/µl Exol and Exolll to the ligation mixture and incubating at 37°C for 30 min followed by heat inactivation of the exonuclease enzymes at 65°C for 10 min. The ligation mixture was mixed with 100 nM color-coded microparticle-coupled amplification primers (amplification primers had 5' biotin, SEQ ID NO: 7, Table 1) mixed with 0.25 mg/ml bovine serum albumin (BSA), 0.5 mM dNTP, 1X phi29 polymerase buffer and 0.25 U/µl phi29 polymerase and the rolling circle amplification was performed at 37°C for 10 min. The RCA mixture was mixed with pre-made oligonucleotides (500 repeats of the sequences in SEQ ID NO: 8-10, Table 1) resulting in instant agglutination.

The results are shown in Fig. 24 showing that the oligonucleotides promoted the labelled RCPs to agglutinate and collapse into a visual, dyed aggregate that could be seen with the naked eye.

The reaction process presented above was repeated using 10, 25 or 40 minutes of RCA but without the last step of mixing the RCA mixture with the pre-made oligonucleotides. In this case, the color-coded microparticles were coupled to detection oligonucleotides having 5' biotin (SEQ ID NO: 11, Table 1). The results are presented in Figs. 23A (10 min RCA), 23B (25 min RCA) and 23C (40 min). As is shown in these figures, even after 40 min of RCA, hardly any visual, dyed aggregates are visible.

The reaction process presented above was repeated. Half of the samples were contacted with oligonucleotides comprising binding regions having a nucleic acid sequence corresponding to at least a portion of the padlock probes and half of the samples were contacted with oligonucleotides comprising binding regions having nucleic sequences not corresponding to at least a portion of the padlock probes. As is shown in Fig. 25, visual, dyed aggregates were only formed in the samples with correct sequence in the pre-made oligonucleotides. This indicates that the present invention has high specificity in producing labelled complexes.

**Table 1 - oligonucleotides**

| Name | Sequence | SEQ ID NO: |
|---|---|---|
| Padlock 1 | TGTACTCACCGGTTCCTATTACGCCGCAACAGCACGCATACTTGAGCTCAT ATGTGGTATTGTCCTGGCAATTCCGG | 1 |
| Padlock 2 | CCGGTTCCGCAGACCCTATTACGCCGCAACAGCACGCATACTTGAGCTCAT ATGTGGTATTGATTCCGGAGTACTCA | 2 |
| Padlock 3 | CAGCACGCACCGGTTCTATTACGCCGCAACAGCACGCATACTTGAGCTCAT ATGTGGTATTGCTCAGCGGCTATAAT | 3 |
| Target 1 | GAACCGGTGAGTACACCGGAATTGCCAGGACGA | 4 |
| Target 2 | GGTCTGCGGAACCGGTGAGTACACCGGAATTAC | 5 |
| Target 3 | GCGGAACCGGTGCGTGCTGATTATAGCCGCTGAGTACACC | 6 |
| Primer | CGAGTATACACC | 7 |
| Oligo 1 repeat | TGTACTCACCGGTTCCTATTACGCCGCAACAGCACGCATACTTGATATTG TCCTGGCAATTCCGG | 8 |
| Oligo 2 repeat | CCGGTTCCGCAGACCCTATTACGCCGCAACAGCACGCATACTTGATATTG ATTCCGGAGTACTCA | 9 |
| Oligo 3 repeat | CAGCACGCACCGGTTCTATTACGCCGCAACAGCACGCATACTTGATATTG CTCAGCGGCTATAAT | 10 |
| Detection oligo | GGTGTATATCTCG | 11 |

The embodiments described above are to be understood as a few illustrative examples of the present invention. In particular, different part solutions in the different embodiments can be combined in other configurations, where technically possible. The scope of the present invention is, however, defined by the appended claims.

## Claims

1. A method for detecting at least one target nucleic acid molecule (1, 1', 1") in a sample, the method comprising:
contacting (S1) the sample with padlock probes (10) comprising at their 5' and 3' ends (14, 15) target-binding regions (12, 13) complementary to probe-binding regions (2, 3) in the at least one target nucleic acid molecule (1, 1', 1");
joining (S2) the 5' and 3' ends (14, 15) of the padlock probes (10) while the target-binding regions (12, 13) are hybridized to the probe-binding regions (2, 3) to form circular padlock probes (10');
rolling circle amplifying (S3) the circular padlock probes (10') with labelled amplification primers (20) comprising a detectable label (22) and a probe-binding region (21) complementary to a primer-binding region (11) of the padlock probes (10) to generate labelled rolling circle products (20');
contacting (S4) the labelled rolling circle products (20') with oligonucleotides (30, 30A) comprising a plurality of binding regions (31) having a nucleic acid sequence corresponding to at least a portion of the padlock probes (10) outside of the primer-binding region (11) to form an agglutinate (50) of labelled complexes (40) between the oligonucleotides (30, 30A) and the labelled rolling circle products (20'); and
detecting (S5) the agglutinate (50) of the labelled complexes (40) to detect the at least one target nucleic acid molecule (1, 1', 1").

2. The method according to claim 1, further comprising removing (S10) any non-joined padlock probes (10) following joining (S2) the 5' and 3' ends (14, 15) of the padlock probes (10) but prior to rolling circle amplifying (S3) the circular padlock probes (10).

3. The method according to claim 1 or 2, wherein joining (S2) the 5' and 3' ends (14, 15) of the padlock probes (10) comprises ligating (S2) the 5' and 3' ends (14, 15) of the padlock probes (10) while the target-binding regions (12, 13) are hybridized to the probe-binding regions (2, 3) to form the circular padlock probes (10').

4. The method according to any one of claims 1 to 3, wherein rolling circle amplifying (S3) comprises rolling circle amplifying (S3) the circular padlock probes (10') with the labelled amplification primers (20) to generate the labelled rolling circle products (20') comprising up to 10 repeats, preferably up to 7 repeats, more preferably up to 5 repeats, and most preferably from 1 up to 5 repeats, of a nucleic acid sequence complementary to a nucleic acid sequence of the padlock probes (10).

5. The method according to any one of claims 1 to 4, wherein contacting (S4) the labelled rolling circle products (20') comprises contacting (S4) the labelled rolling circle products (20') with the oligonucleotides (30, 30A) comprising at least 100 repeats, preferably at least 250 repeats, more preferably at least 500 repeats, and most preferably from 500 up to 1500 repeats, of the binding region (31).

6. The method according to any one of claims 1 to 5, wherein rolling circle amplifying (S3) comprises rolling circle amplifying (S3) the circular padlock probes (10) with the labelled amplification primers (20) comprising a detectable bead (22) and the probe-binding region (21) to generate bead-labelled rolling circle products (20').

7. The method according to any one of claims 1 to 6, wherein contacting (S4) the labelled rolling circle products (20') comprises contacting (S4) the labelled rolling circle products (20') with circular oligonucleotides (30A) comprising the plurality of binding regions (31) to form the agglutinate (50) of the labelled complexes (40) between the circular oligonucleotides (30A) and the labelled rolling circle products (20').

8. The method according to any one of claims 1 to 7, wherein
rolling circle amplifying (S3) comprises rolling circle amplifying (S3) the circular padlock probes (10') with multiple sets of labelled amplification primers (20, 20A) to generate the labelled rolling circle products (20');
the labelled amplification primers (20, 20A) in each set of the multiple sets comprise the same probe-binding region (21, 21A);
the probe-binding region (21) of the labelled amplification primers (20) in one set of the multiple sets is complementary to another primer-binding region (11) of the padlock probes (10) than the primer-binding region (21A) of the labelled amplification primers (20A) in another set of the multiple sets; and
the labelled amplification primers (20, 20A) in the multiple sets have the same detectable label (22).

9. The method according to any one of claims 1 to 8, wherein
contacting (S1) the sample with the padlock probes (10) comprises contacting (S1) the sample with multiple sets of padlock probes (10, 10A), wherein
the padlock probes (10, 10A) in each set of the multiple sets comprise same target-binding regions (12, 13, 12A, 13A); and
the target-binding regions (12, 13) of the padlock probes (10) in one set of the multiple sets are complementary to other probe-binding regions (2, 3) in the at least one target nucleic acid molecule (1, 1', 1") than the target-binding regions (12A, 13A) of the padlock probes (10A) in another set of the multiple sets;
rolling circle amplification (S3) comprises rolling circle amplifying (S3) the circular padlock probes (10', 10A') with multiple sets of labelled amplification primers (20, 20A) to generate multiple sets of labelled rolling circle products (20'), wherein
the labelled amplification primers (20, 20A) in each set of the multiple sets comprise the same probe-binding region (21, 21A);
the probe-binding region (21) of the labelled amplification primers (20) in one set of the multiple sets are complementary to another primer-binding region (11) of the padlock probes (10, 10A) than the primer-binding region (21A) of the labelled amplification primers (20A) in another set of the multiple sets; and
the labelled amplification primers (20, 20A) in the multiple sets have the same detectable label (22).

10. The method according to any one of claims 1 to 8, wherein
contacting (S1) the sample with the padlock probes (10) comprises contacting (S1) the sample with multiple sets of padlock probes (10, 10A), wherein
the padlock probes (10, 10A) in each set of the multiple sets comprise same target-binding regions (12, 13, 12A, 13A); and
the target-binding regions (12, 13) of the padlock probes (10) in one set of the multiple sets are complementary to other probe-binding regions (2, 3) in the at least one target nucleic acid molecule (1, 1', 1") than the target-binding regions (12A, 13A) of the padlock probes (10A) in another set of the multiple sets; and
the padlock probes (10, 10A) in the multiple sets comprise the same primer-binding region (11).

11. The method according to any one of claims 1 to 10, wherein
contacting (S1) the sample comprises contacting (S1) a sample comprising multiple different target nucleic acid molecules (1, 1B) with multiple sets of padlock probes (10, 10B), wherein
the padlock probes (10, 10B) in each set of the multiple sets comprise the same target-binding regions (12, 13, 12B, 13B); and
the target-binding regions (12, 13) of the padlock probes (10) in one set of the multiple sets are complementary to other probe-binding regions (2, 3) in the multiple different target nucleic acid molecules (1, 1B) than the target-binding regions (12B, 13B) of the padlock probes (10B) in another set of the multiple sets;
rolling circle amplifying (S3) comprises rolling circle amplifying (S3) the circular padlock probes (10', 10B') with multiple sets of labelled amplification primers (20, 20B) to generate the labelled rolling circle products (20', 20B'), wherein
the labelled amplification primers (20, 20B) in each set of the multiple sets comprise the same probe-binding region (21, 21B) and the same detectable label (22, 22B);
the probe-binding region (21) of the labelled amplification primers (20) in one set of the multiple sets is complementary to another primer-binding region (11) of the padlock probes (10, 10B) than the primer-binding region (21B) of the labelled amplification primers (20B) in another set of the multiple sets; and
the detectable label (22) of the labelled amplification primers (20) in one set of the multiple sets is different than the detectable label (22B) of the labelled amplification primers (20B) in another set of the multiple sets;
contacting (S4) the labelled rolling circle products (20') comprises contacting (S4) the labelled rolling circle products (20', 20B') with multiple sets of oligonucleotides (30, 30B) to form at least one gglutinate (50) of the labelled complexes (40, 40B) between the oligonucleotides (30, 30B) and the labelled rolling circle products (20', 20B'), wherein
the oligonucleotides (30, 30B) in each set of the multiple sets comprise the same binding regions (31, 31B); and
the binding regions (31) of the oligonucleotides (30) in one set of the multiple sets have a different nucleic acid sequence than the binding regions (31B) of the oligonucleotides (30B) in another set of the multiple sets; and
detecting (S5) the agglutinate of the labelled complexes (40) comprises detecting (S5) the at least one agglutinate (50) of the multiple labelled complexes (40, 40B) with different labels (22, 22B) to detect the multiple target nucleic acid molecules (1, 1B).

12. The method according to any one of claims 1 to 11, wherein
contacting (S1) the sample comprises contacting (S1) the sample with multiple target-binding molecules (72, 74) binding specifically to a target molecule (70), and padlock probes (10), wherein
each target-binding molecule (72, 74) of the multiple target-binding molecules (72, 74) comprise a respective target nucleic acid molecule (1', 1");
the padlock probes (10) comprise at their 5' and 3' ends (14, 15) target-binding regions (12, 13) complementary to probe-binding regions (2, 3) in the respective target nucleic acid molecules (1', 1");
joining (S2) the 5' and 3' ends (14, 15) of the padlock probes (10) comprises joining (S2) the 5' and 3' ends (14, 15) of the padlock probes (10) while the target-binding regions (12, 13) are hybridized to the probe-binding regions (2, 3) and the multiple target-binding molecules (72, 74) are binding to the target molecule (70) to form circular padlock probes (10'); and
detecting the agglutinate (50) of the labelled complexes (40) comprises detecting (S5) the agglutinate (50) of the labelled complexes (40) to detect the respective target nucleic acid molecules (1', 1") and thereby the target molecule (70).

13. A kit for detecting at least one target nucleic acid molecule (1, 1', 1") in a sample, the kit comprising:
padlock probes (10) comprising at their 5' and 3' ends (14, 15) target-binding regions (12, 13) complementary to probe-binding regions (2, 3) in at least one target nucleic acid molecule (1, 1', 1");
labelled amplification primers (20) comprising a detectable label (22) and a probe-binding region (21) complementary to a primer-binding region (11) of the padlock probes (10);
oligonucleotides (30, 30A) comprising a plurality of binding regions (31) having a nucleic acid sequence corresponding to at least a portion of the padlock probes (10) outside of the primer-binding region (11);
optionally a ligase adapted to ligate the 5' and 3' ends (14, 15) of the padlock probes (10) while the target-binding regions (12, 13) are hybridized to the probe-binding regions (2, 3) to form circular padlock probes (10'); and
optionally a polymerase adapted to rolling circle amplify the circular padlock probes (10') with the labelled amplification primers (20) to generate labelled rolling circle products (20').

14. A microfluidic chip (100) comprising:
a sample input (110) configured to receive a sample comprising at least one target nucleic acid molecule (1, 1', 1")
a microfluidic channel (140) in fluid connection with the sample input (110) and comprising:
a reagent section (141) comprising i) padlock probes (10) comprising at their 5' and 3' ends (14, 15) target-binding regions (12, 13) complementary to probe-binding regions (2, 3) in the at least one target nucleic acid molecule (1, 1', 1"), ii) a ligase adapted to ligate the 5' and 3' ends (14, 15) of the padlock probes (10) while the target-binding regions (12, 13) are hybridized to the probe-binding regions (2, 3) to form circular padlock probes (10'), iii) labelled amplification primers (20) comprising a detectable label (22) and a probe-binding region (21) complementary to a primer-binding region (11) of the padlock probes (10), and iv) a polymerase adapted to rolling circle amplify the circular padlock probes (10') with the labelled amplification primers (20) to generate labelled rolling circle products (20'); and
a detection section (142) comprising oligonucleotides (30, 30A) comprising a plurality of binding regions (31) having a nucleic acid sequence corresponding to at least a portion of the padlock probes (10) outside of the primer-binding region (11); and
a detection window (120, 122, 124) aligned with at least a portion of the detection section (142) and arranged to enable visual access to the at least a portion of the detection section (142).

15. The microfluidic chip according to claim 14, wherein
the microfluidic channel (140) also comprises a control section (143) comprising oligonucleotides comprising a plurality of binding regions having a nucleic acid sequence complementary to the probe-binding region (21) of the labelled amplification primers (20); and
the microfluidic chip (100) further comprises a control window (130) aligned with the control section (143) and arranged to enable visual access to the control section (143).

## Patentansprüche

1. Verfahren zum Nachweis mindestens eines Zielnukleinsäuremoleküls (1, 1', 1'') in einer Probe, wobei das Verfahren Folgendes umfasst:
Kontaktieren (S1) der Probe mit Padlock-Sonden (10), die an ihren 5'- und 3'-Enden (14, 15) Zielbindungsregionen (12, 13) umfassen, die zu Sondenbindungsregionen (2, 3) in dem mindestens einen Zielnukleinsäuremolekül (1, 1', 1") komplementär sind;
Verbinden (S2) der 5'- und 3'-Enden (14, 15) der Padlock-Sonden (10), während die Zielbindungsregionen (12, 13) mit den Sondenbindungsregionen (2, 3) hybridisiert werden, um zirkuläre Padlock-Sonden (10') zu bilden;
Rolling-Circle-Amplifizieren (S3) der zirkulären Padlock-Sonden (10') mit markierten Amplifikationsprimern (20), die eine nachweisbare Markierung (22) und eine Sondenbindungsregion (21) umfassen, die zu einer Primerbindungsregion (11) der Padlock-Sonden (10) komplementär ist, um markierte Rolling-Circle-Produkte (20') zu erzeugen;
Kontaktieren (S4) der markierten Rolling-Circle-Produkte (20') mit Oligonukleotiden (30, 30A), die eine Vielzahl von Bindungsregionen (31) mit einer Nukleinsäuresequenz umfassen, die mindestens einem Teil der Padlock-Sonden (10) außerhalb der Primerbindungsregion (11) entspricht, um ein Agglutinat (50) von markierten Komplexen (40) zwischen den Oligonukleotiden (30, 30A) und den markierten Rolling-Circle-Produkten (20) zu bilden; und
Nachweisen (S5) des Agglutinats (50) der markierten Komplexe (40) zum Nachweisen des mindestens einen Zielnukleinsäuremoleküls (1, 1', 1'').

2. Verfahren nach Anspruch 1, ferner umfassend das Entfernen (S10) jeglicher nicht verbundener Padlock-Sonden (10) nach dem Verbinden (S2) der 5'- und 3'-Enden (14, 15) der Padlock-Sonden (10), jedoch vor dem Rolling-Circle-Amplifizieren (S3) der zirkulären Padlock-Sonden (10).

3. Verfahren nach Anspruch 1 oder 2, wobei das Verbinden (S2) der 5'- und 3'-Enden (14, 15) der Padlock-Sonden (10) das Ligieren (S2) der 5'- und 3'-Enden (14, 15) der Padlock-Sonden (10) umfasst, während die Zielbindungsregionen (12, 13) mit den Sondenbindungsregionen (2, 3) hybridisiert werden, um die zirkulären Padlock-Sonden (10') zu bilden.

4. Verfahren nach einem der Ansprüche 1 bis 3, wobei das Rolling-Circle-Amplifizieren (S3) das Rolling-Circle-Amplifizieren (S3) der zirkulären Padlock-Sonden (10') mit den markierten Amplifikationsprimern (20) umfasst, um die markierten Rolling-Circle-Produkte (20') zu erzeugen, die bis zu 10 Wiederholungen, bevorzugt bis zu 7 Wiederholungen, bevorzugter bis zu 5 Wiederholungen und am meisten bevorzugt von 1 bis zu 5 Wiederholungen einer Nukleinsäuresequenz umfassen, die zu einer Nukleinsäuresequenz der Padlock-Sonden (10) komplementär ist.

5. Verfahren nach einem der Ansprüche 1 bis 4, wobei das Kontaktieren (S4) der markierten Rolling-Circle-Produkte (20') das Kontaktieren (S4) der markierten Rolling-Circle-Produkte (20') mit den Oligonukleotiden (30, 30A) umfasst, die mindestens 100 Wiederholungen, bevorzugt mindestens 250 Wiederholungen, bevorzugter mindestens 500 Wiederholungen und am meisten bevorzugt von 500 bis 1500 Wiederholungen der Bindungsregion (31) umfassen.

6. Verfahren nach einem der Ansprüche 1 bis 5, wobei das Rolling-Circle-Amplifizieren (S3) ein Rolling-Circle-Amplifizieren (S3) der zirkulären Padlock-Sonden (10) mit den markierten Amplifikationsprimern (20) umfasst, die einen nachweisbaren Bead (22) und die Sondenbindungsregion (21) umfassen, um Bead-markierte Rolling-Circle-Produkte (20') zu erzeugen.

7. Verfahren nach einem der Ansprüche 1 bis 6, wobei das Kontaktieren (S4) der markierten Rolling-Circle-Produkte (20') das Kontaktieren (S4) der markierten Rolling-Circle-Produkte (20') mit zirkulären Oligonukleotiden (30A) umfasst, die die Vielzahl von Bindungsregionen (31) umfassen, um das Agglutinat (50) der markierten Komplexe (40) zwischen den zirkulären Oligonukleotiden (30A) und den markierten Rolling-Circle-Produkten (20) zu bilden.

8. Verfahren nach einem der Ansprüche 1 bis 7, wobei
das Rolling-Circle-Amplifizieren (S3) das Rolling-Circle-Amplifizieren (S3) der zirkulären Padlock-Sonden (10') mit mehreren Sätzen markierter Amplifikationsprimern (20, 20A) umfasst, um die markierten Rolling-Circle-Produkte (20) zu erzeugen;
die markierten Amplifikationsprimern (20, 20A) in jedem Satz der mehreren Sätze die gleiche Sondenbindungsregion (21, 21A) umfassen; die Sondenbindungsregion (21) der markierten Amplifikationsprimern (20) in einem Satz der mehreren Sätze komplementär zu einer anderen Primerbindungsregion (11) der Padlock-Sonden (10) ist als die Primerbindungsregion (21A) der markierten Amplifikationsprimern (20A) in einem anderen Satz der mehreren Sätze; und
die markierten Amplifikationsprimern (20, 20A) in den mehreren Sätzen die gleiche nachweisbare Markierung (22) aufweisen.

9. Verfahren nach einem der Ansprüche 1 bis 8, wobei
das Kontaktieren (S1) der Probe mit den Padlock-Sonden (10) das Kontaktieren (S1) der Probe mit mehreren Sätzen von Padlock-Sonden (10, 10A) umfasst, wobei
die Padlock-Sonden (10, 10A) in jedem Satz der mehreren Sätze die gleichen Zielbindungsregionen (12, 13, 12A, 13A) umfassen; und
die Zielbindungsregionen (12, 13) der Padlock-Sonden (10) in einem Satz der mehreren Sätze komplementär zu anderen Sondenbindungsregionen (2, 3) in dem mindestens einen Zielnukleinsäuremolekül (1, 1', 1") sind als die Zielbindungsregionen (12A, 13A) der Padlock-Sonden (10A) in einem anderen Satz der mehreren Sätze;
die Rolling-Circle-Amplifikation (S3) ein Rolling-Circle-Amplifizieren (S3) der zirkulären Padlock-Sonden (10', 10A') mit mehreren Sätzen markierter Amplifikationsprimern (20, 20A) umfasst, um mehrere Sätze markierter Rolling-Circle-Produkte (20') zu erzeugen, wobei
die markierten Amplifikationsprimern (20, 20A) in jedem Satz der mehreren Sätze die gleiche Sondenbindungsregion (21, 21A) umfassen; die Sondenbindungsregion (21) der markierten Amplifikationsprimern (20) in einem Satz der mehreren Sätze komplementär zu einer anderen Primerbindungsregion (11) der Padlock-Sonden (10, 10A) ist als die Primerbindungsregion (21A) der markierten Amplifikationsprimern (20A) in einem anderen Satz der mehreren Sätze; und
die markierten Amplifikationsprimern (20, 20A) in den mehreren Sätzen die gleiche nachweisbare Markierung (22) aufweisen.

10. Verfahren nach einem der Ansprüche 1 bis 8, wobei
das Kontaktieren (S1) der Probe mit den Padlock-Sonden (10) das Kontaktieren (S1) der Probe mit mehreren Sätzen von Padlock-Sonden (10, 10A) umfasst, wobei
die Padlock-Sonden (10, 10A) in jedem Satz der mehreren Sätze die gleichen Zielbindungsregionen (12, 13, 12A, 13A) umfassen; und die Zielbindungsregionen (12, 13) der Padlock-Sonden (10) in einem Satz der mehreren Sätze komplementär zu anderen Sondenbindungsregionen (2, 3) in dem mindestens einen Zielnukleinsäuremolekül (1, 1', 1") sind als die Zielbindungsregionen (12A, 13A) der Padlock-Sonden (10A) in einem anderen Satz der mehreren Sätze; und
die Padlock-Sonden (10, 10A) in den mehreren Sätzen die gleiche Primerbindungsregion (11) umfassen.

11. Verfahren nach einem der Ansprüche 1 bis 10, wobei
das Kontaktieren (S1) der Probe das Kontaktieren (S1) einer Probe, die mehrere verschiedene Zielnukleinsäuremoleküle (1, 1B) umfasst, mit mehreren Sätzen von Padlock-Sonden (10, 10B) umfasst, wobei die Padlock-Sonden (10, 10B) in jedem Satz der mehreren Sätze die gleichen Zielbindungsregionen (12, 13, 12B, 13B) umfassen; und die Zielbindungsregionen (12, 13) der Padlock-Sonden (10) in einem Satz der mehreren Sätze komplementär zu anderen Sondenbindungsregionen (2, 3) in den mehreren verschiedenen Zielnukleinsäuremolekülen (1, 1B) sind als die Zielbindungsregionen (12B, 13B) der Padlock-Sonden (10B) in einem anderen Satz der mehreren Sätze;
das Rolling-Circle-Amplifizieren (S3) das Rolling-Circle-Amplifizieren (S3) der zirkulären Padlock-Sonden (10, 10B') mit mehreren Sätzen markierter Amplifikationsprimern (20, 20B) umfasst, um die markierten Rolling-Circle-Produkte (20, 20B') zu erzeugen, wobei
die markierten Amplifikationsprimern (20, 20B) in jedem Satz der mehreren Sätze die gleiche Sondenbindungsregion (21, 21B) und die gleiche nachweisbare Markierung (22, 22B) umfassen;
die Sondenbindungsregion (21) der markierten Amplifikationsprimern (20) in einem Satz der mehreren Sätze komplementär zu einer anderen Primerbindungsregion (11) der Padlock-Sonden (10, 10B) ist als die Primerbindungsregion (21B) der markierten Amplifikationsprimern (20B) in einem anderen Satz der mehreren Sätze; und
sich die nachweisbare Markierung (22) der markierten Amplifikationsprimern (20) in einem Satz der mehreren Sätze von der nachweisbaren Markierung (22B) der markierten Amplifikationsprimern (20B) in einem anderen Satz der mehreren Sätze unterscheidet;
das Kontaktieren (S4) der markierten Rolling-Circle-Produkte (20') das Kontaktieren (S4) der markierten Rolling-Circle-Produkte (20', 20B') mit mehreren Sätzen von Oligonukleotiden (30, 30B) umfasst, um mindestens ein Agglutinat (50) der markierten Komplexe (40, 40B) zwischen den Oligonukleotiden (30, 30B) und den markierten Rolling-Circle-Produkten (20', 20B') zu bilden, wobei
die Oligonukleotide (30, 30B) in jedem Satz der mehreren Sätze die gleichen Bindungsregionen (31, 31B) umfassen; und
die Bindungsregionen (31) der Oligonukleotide (30) in einem Satz der mehreren Sätze eine andere Nukleinsäuresequenz aufweisen als die Bindungsregionen (31B) der Oligonukleotide (30B) in einem anderen Satz der mehreren Sätze; und
der Nachweis (S5) des Agglutinats der markierten Komplexe (40) den Nachweis (S5) des mindestens einen Agglutinats (50) der mehreren markierten Komplexe (40, 40B) mit verschiedenen Markierungen (22, 22B) zum Nachweis der mehreren Zielnukleinsäuremoleküle (1, 1B) umfasst.

12. Verfahren nach einem der Ansprüche 1 bis 11, wobei
das Kontaktieren (S1) der Probe das Kontaktieren (S1) der Probe mit mehreren Zielbindungsmolekülen (72, 74), die spezifisch an ein Zielmolekül (70) binden, und Padlock-Sonden (10) umfasst, wobei jedes Zielbindungsmolekül (72, 74) der mehreren Zielbindungsmoleküle (72, 74) ein jeweiliges Zielnukleinsäuremolekül (1', 1") umfasst;
die Padlock-Sonden (10) an ihren 5'- und 3'-Enden (14, 15) Zielbindungsregionen (12, 13) umfassen, die zu Sondenbindungsregionen (2, 3) in den jeweiligen Zielnukleinsäuremolekülen (1', 1") komplementär sind;
das Verbinden (S2) der 5'- und 3'-Enden (14, 15) der Padlock-Sonden (10) das Verbinden (S2) der 5'- und 3'-Enden (14, 15) der Padlock-Sonden (10) umfasst, während die Zielbindungsregionen (12, 13) mit den Sondenbindungsregionen (2, 3) hybridisiert werden und die mehreren Zielbindungsmoleküle (72, 74) an das Zielmolekül (70) binden, um zirkuläre Padlock-Sonden (10') zu bilden; und
der Nachweis des Agglutinats (50) der markierten Komplexe (40) den Nachweis (S5) des Agglutinats (50) der markierten Komplexe (40) zum Nachweis der jeweiligen Zielnukleinsäuremoleküle (1', 1") und damit des Zielmoleküls (70) umfasst.

13. Kit zum Nachweis mindestens eines Zielnukleinsäuremoleküls (1, 1', 1") in einer Probe, wobei das Kit Folgendes umfasst:
Padlock-Sonden (10), die an ihren 5'- und 3'-Enden (14, 15) Zielbindungsregionen (12, 13) aufweisen, die zu Sondenbindungsregionen (2, 3) in mindestens einem Zielnukleinsäuremolekül (1, 1', 1") komplementär sind;
markierte Amplifikationsprimern (20), die eine nachweisbare Markierung (22) und eine Sondenbindungsregion (21) umfassen, die zu einer Primerbindungsregion (11) der Padlock-Sonden (10) komplementär ist;
Oligonukleotide (30, 30A), umfassend eine Vielzahl von Bindungsregionen (31) mit einer Nukleinsäuresequenz, die mindestens einem Teil der Padlock-Sonden (10) außerhalb der Primerbindungsregion (11) entspricht;
optional eine Ligase, die angepasst ist, um die 5'- und 3'-Enden (14, 15) der Padlock-Sonden (10) zu ligieren, während die Zielbindungsregionen (12, 13) an die Sondenbindungsregionen (2, 3) hybridisiert werden, um zirkuläre Padlock-Sonden (10') zu bilden; und
optional eine Polymerase, die zum Rolling-Circle-Amplifizieren der zirkulären Padlock-Sonden (10') mit den markierten Amplifikationsprimern (20) angepasst ist, um markierte Rolling Circle-Produkte (20) zu erzeugen.

14. Mikrofluidischer Chip (100), Folgendes umfassend:
einen Probeneingang (110), der konfiguriert ist, um eine Probe aufzunehmen, die mindestens ein Zielnukleinsäuremolekül (1, 1', 1'') umfasst,
einen mikrofluidischen Kanal (140), der in Fluidverbindung mit dem Probeneingang (110) steht und Folgendes umfasst:
einen Reagenzienabschnitt (141), umfassend i) Padlock-Sonden (10), die an ihren 5'- und 3'-Enden (14, 15) Zielbindungsregionen (12, 13) umfassen, die zu Sondenbindungsregionen (2, 3) in dem mindestens einen Zielnukleinsäuremolekül (1, 1', 1") komplementär sind, ii) eine Ligase, die angepasst ist, um die 5'- und 3'-Enden (14, 15) der Padlock-Sonden (10) zu ligieren, während die Zielbindungsregionen (12, 13) an die Sondenbindungsregionen (2, 3) hybridisiert werden, um zirkuläre Padlock-Sonden (10') zu bilden, iii) markierte Amplifikationsprimern (20), die eine nachweisbare Markierung (22) und eine Sondenbindungsregion (21) umfassen, die zu einer Primerbindungsregion (11) der Padlock-Sonden (10) komplementär ist, und iv) eine Polymerase, die zum Rolling-Circle-Amplifizieren der zirkulären Padlock-Sonden (10') mit den markierten Amplifikationsprimern (20) angepasst ist, um markierte Rolling Circle-Produkte (20) zu erzeugen; und
einen Nachweisabschnitt (142), umfassend Oligonukleotide (30, 30A), umfassend eine Vielzahl von Bindungsregionen (31) mit einer Nukleinsäuresequenz, die mindestens einem Teil der Padlock-Sonden (10) außerhalb der Primerbindungsregion (11) entspricht; und
ein Nachweisfenster (120, 122, 124), das an mindestens einem Teil des Nachweisabschnitts (142) ausgerichtet und angeordnet ist, um einen visuellen Zugang zu dem mindestens einen Teil des Erfassungsabschnitts (142) zu ermöglichen.

15. Mikrofluidischer Chip nach Anspruch 14, wobei
der mikrofluidische Kanal (140) auch einen Kontrollabschnitt (143) umfasst, der Oligonukleotide umfasst, die eine Vielzahl von Bindungsregionen mit einer Nukleinsäuresequenz umfassen, die zu der Probenbindungsregion (21) der markierten Amplifikationsprimer (20) komplementär ist; und
der mikrofluidische Chip (100) ferner ein Kontrollfenster (130) umfasst, das an dem Kontrollabschnitt (143) ausgerichtet und angeordnet ist, um einen visuellen Zugang zu dem Kontrollabschnitt (143) zu ermöglichen.

## Revendications

1. Procédé de détection d'au moins une molécule d'acide nucléique cible (1, 1', 1'') dans un échantillon, le procédé comprenant :
la mise en contact (S1) de l'échantillon avec des sondes cadenas (10) comprenant à leurs extrémités 5' et 3' (14, 15) des régions de liaison à la cible (12, 13) complémentaires des régions de liaison à la sonde (2, 3) dans l'au moins une molécule d'acide nucléique cible (1, 1', 1") ;
la jonction (S2) des extrémités 5' et 3' (14, 15) des sondes cadenas (10) tandis que les régions de liaison à la cible (12, 13) sont hybridées aux régions de liaison à la sonde (2, 3) pour former des sondes cadenas circulaires (10') ;
l'amplification en cercle roulant (S3) des sondes cadenas circulaires (10') avec des amorces d'amplification marquées (20) comprenant un marqueur détectable (22) et une région de liaison à la sonde (21) complémentaire d'une région de liaison à l'amorce (11) des sondes cadenas (10) pour générer des produits de cercle roulant marqués (20') ;
la mise en contact (S4) des produits de cercle roulant marqués (20') avec des oligonucléotides (30, 30A) comprenant une pluralité de régions de liaison (31) ayant une séquence d'acide nucléique correspondant à au moins une partie des sondes cadenas (10) à l'extérieur de la région de liaison à l'amorce (11) pour former un agglutinat (50) de complexes marqués (40) entre les oligonucléotides (30, 30A) et les produits de cercle roulant marqués (20') ; et
la détection (S5) de l'agglutinat (50) des complexes marqués (40) pour détecter au moins une molécule d'acide nucléique cible (1, 1', 1").

2. Procédé selon la revendication 1, comprenant en outre le retrait (S10) de toutes les sondes cadenas non jointes (10) après la jonction (S2) des extrémités 5' et 3' (14, 15) des sondes cadenas (10) mais avant l'amplification en cercle roulant (S3) des sondes cadenas circulaires (10).

3. Procédé selon la revendication 1 ou 2, dans lequel la jonction (S2) des extrémités 5' et 3' (14, 15) des sondes cadenas (10) comprend la ligature (S2) des extrémités 5' et 3' (14, 15) des sondes cadenas (10) tandis que les régions de liaison à la cible (12, 13) sont hybridées aux régions de liaison à la sonde (2, 3) pour former les sondes cadenas circulaires (10').

4. Procédé selon l'une quelconque des revendications 1 à 3, dans lequel l'amplification en cercle roulant (S3) comprend l'amplification en cercle roulant (S3) des sondes cadenas circulaires (10') avec les amorces d'amplification marquées (20) pour générer les produits de cercle roulant marqués (20') comprenant jusqu'à 10 répétitions, préférablement jusqu'à 7 répétitions, plus préférablement jusqu'à 5 répétitions, et le plus préférablement de 1 à 5 répétitions, d'une séquence d'acide nucléique complémentaire d'une séquence d'acide nucléique des sondes cadenas (10).

5. Procédé selon l'une quelconque des revendications 1 à 4, dans lequel la mise en contact (S4) des produits de cercle roulant marqués (20') comprend la mise en contact (S4) des produits de cercle roulant marqués (20') avec les oligonucléotides (30, 30A) comprenant au moins 100 répétitions, préférablement au moins 250 répétitions, plus préférablement au moins 500 répétitions, et le plus préférablement de 500 à 1500 répétitions, de la région de liaison (31).

6. Procédé selon l'une quelconque des revendications 1 à 5, dans lequel l'amplification en cercle roulant (S3) comprend l'amplification en cercle roulant (S3) des sondes cadenas circulaires (10) avec les amorces d'amplification marquées (20) comprenant une bille détectable (22) et la région de liaison à la sonde (21) pour générer des produits de cercle roulant marqués par bille (20').

7. Procédé selon l'une quelconque des revendications 1 à 6, dans lequel la mise en contact (S4) des produits de cercle roulant marqués (20') comprend la mise en contact (S4) des produits de cercle roulant marqués (20') avec des oligonucléotides circulaires (30A) comprenant la pluralité de régions de liaison (31) pour former l'agglutinat (50) des complexes marqués (40) entre les oligonucléotides circulaires (30A) et les produits de cercle roulant marqués (20').

8. Procédé selon l'une quelconque des revendications 1 à 7, dans lequel
l'amplification en cercle roulant (S3) comprend l'amplification en cercle roulant (S3) des sondes cadenas circulaires (10') avec multiples ensembles d'amorces d'amplification marquées (20, 20A) pour générer les produits de cercle roulant marqués (20') ;
les amorces d'amplification marquées (20, 20A) dans chaque ensemble des multiples ensembles comprennent la même région de liaison à la sonde (21, 21A) ;
la région de liaison à la sonde (21) des amorces d'amplification marquées (20) dans un ensemble des multiples ensembles est complémentaire d'une autre région de liaison à l'amorce (11) des sondes cadenas (10) que de la région de liaison à l'amorce (21A) des amorces d'amplification marquées (20A) dans un autre ensemble des multiples ensembles ; et
les amorces d'amplification marquées (20, 20A) dans les multiples ensembles ont le même marqueur détectable (22).

9. Procédé selon l'une quelconque des revendications 1 à 8, dans lequel
la mise en contact (S1) de l'échantillon avec les sondes cadenas (10) comprend la mise en contact (S1) de l'échantillon avec multiples ensembles de sondes cadenas (10, 10A), dans lequel
les sondes cadenas (10, 10A) de chaque ensemble des multiples ensembles comprennent les mêmes régions de liaison à la cible (12, 13, 12A, 13A) ; et
les régions de liaison à la cible (12, 13) des sondes cadenas (10) dans un ensemble des multiples ensembles sont complémentaires d'autres régions de liaison à la sonde (2, 3) dans l'au moins une molécule d'acide nucléique cible (1, 1', 1") que les régions de liaison à la cible (12A, 13A) des sondes cadenas (10A) dans un autre ensemble des multiples ensembles ;
l'amplification en cercle roulant (S3) comprend l'amplification en cercle roulant (S3) des sondes cadenas circulaires (10', 10A') avec multiples ensembles d'amorces d'amplification marquées (20, 20A) pour générer multiples ensembles de produits de cercle roulant marqués (20'), dans lequel
les amorces d'amplification marquées (20, 20A) dans chaque ensemble des multiples ensembles comprennent la même région de liaison à la sonde (21, 21A) ;
la région de liaison à la sonde (21) des amorces d'amplification marquées (20) dans un ensemble des multiples ensembles est complémentaire d'une autre région de liaison à l'amorce (11) des sondes cadenas (10, 10A) que de la région de liaison à l'amorce (21A) des amorces d'amplification marquées (20A) dans un autre ensemble des multiples ensembles ; et
les amorces d'amplification marquées (20, 20A) dans les multiples ensembles ont le même marqueur détectable (22).

10. Procédé selon l'une quelconque des revendications 1 à 8, dans lequel
la mise en contact (S1) de l'échantillon avec les sondes cadenas (10) comprend la mise en contact (S1) de l'échantillon avec multiples ensembles de sondes cadenas (10, 10A), dans lequel
les sondes cadenas (10, 10A) de chaque ensemble des multiples ensembles comprennent les mêmes régions de liaison à la cible (12, 13, 12A, 13A) ; et
les régions de liaison à la cible (12, 13) des sondes cadenas (10) dans un ensemble des multiples ensembles sont complémentaires d'autres régions de liaison à la sonde (2, 3) dans l'au moins une molécule d'acide nucléique cible (1, 1', 1") que les régions de liaison à la cible (12A, 13A) des sondes cadenas (10A) dans un autre ensemble des multiples ensembles ; et
les sondes cadenas (10, 10A) dans les multiples ensembles comprennent la même région de liaison à l'amorce (11).

11. Procédé selon l'une quelconque des revendications 1 à 10, dans lequel
la mise en contact (S1) de l'échantillon comprend la mise en contact (S1) d'un échantillon comprenant multiples molécules d'acide nucléique cible différentes (1, 1B) avec multiples ensembles de sondes cadenas (10, 10B), dans lequel
les sondes cadenas (10, 10B) de chaque ensemble des multiples ensembles comprennent les mêmes régions de liaison à la cible (12, 13, 12B, 13B) ; et
les régions de liaison à la cible (12, 13) des sondes cadenas (10) dans un ensemble des multiples ensembles sont complémentaires à d'autres régions de liaison à la sonde (2, 3) dans les multiples molécules d'acide nucléique cible différentes (1, 1B) que les régions de liaison à la cible (12B, 13B) des sondes cadenas (10B) dans un autre ensemble des multiples ensembles ;
l'amplification en cercle roulant (S3) comprend l'amplification en cercle roulant (S3) des sondes cadenas circulaires (10', 10B') avec multiples ensembles d'amorces d'amplification marquées (20, 20B) pour générer les produits de cercle roulant marqués (20', 20B'), dans lequel
les amorces d'amplification marquées (20, 20B) dans chaque ensemble des multiples ensembles comprennent la même région de liaison à la sonde (21, 21B) et le même marqueur détectable (22, 22B) ;
la région de liaison à la sonde (21) des amorces d'amplification marquées (20) dans un ensemble des multiples ensembles est complémentaire d'une autre région de liaison à l'amorce (11) des sondes cadenas (10, 10B) que la région de liaison à l'amorce (21B) des amorces d'amplification marquées (20B) dans un autre ensemble des multiples ensembles ; et
le marqueur détectable (22) des amorces d'amplification marquées (20) dans un ensemble des multiples ensembles est différent du marqueur détectable (22B) des amorces d'amplification marquées (20B) dans un autre ensemble des multiples ensembles ;
la mise en contact (S4) des produits de cercle roulant marqués (20') comprend la mise en contact (S4) des produits de cercle roulant marqués (20', 20B') avec multiples ensembles d'oligonucléotides (30, 30B) pour former au moins un agglutinat (50) des complexes marqués (40, 40B) entre les oligonucléotides (30, 30B) et les produits de cercle roulant marqués (20', 20B'), dans lequel
les oligonucléotides (30, 30B) de chaque ensemble des multiples ensembles comprennent les mêmes régions de liaison (31, 31B) ; et
les régions de liaison (31) des oligonucléotides (30) dans un ensemble des multiples ensembles ont une séquence d'acide nucléique différente de celle des régions de liaison (31B) des oligonucléotides (30B) dans un autre ensemble des multiples ensembles ; et
la détection (S5) de l'agglutinat des complexes marqués (40) comprend la détection (S5) de l'au moins un agglutinat (50) des multiples complexes marqués (40, 40B) avec différents marqueurs (22, 22B) pour détecter les multiples molécules d'acide nucléique cibles (1, 1B).

12. Procédé selon l'une quelconque des revendications 1 à 11, dans lequel
la mise en contact (S1) de l'échantillon comprend la mise en contact (S1) de l'échantillon avec multiples molécules de liaison à la cible (72, 74) se liant spécifiquement à une molécule cible (70), et des sondes cadenas (10), dans lequel chaque molécule de liaison à la cible (72, 74) des multiples molécules de liaison à la cible (72, 74) comprend une molécule d'acide nucléique cible respective (1', 1") ;
les sondes cadenas (10) comprennent à leurs extrémités 5' et 3' (14, 15) des régions de liaison à la cible (12, 13) complémentaires des régions de liaison à la sonde (2, 3) dans les molécules d'acide nucléique cibles respectives (1', 1") ; la jonction (S2) des extrémités 5' et 3' (14, 15) des sondes cadenas (10) comprend la jonction (S2) des extrémités 5' et 3' (14, 15) des sondes cadenas (10) tandis que les régions de liaison à la cible (12, 13) sont hybridées aux régions de liaison à la sonde (2, 3) et les multiples molécules de liaison à la cible (72, 74) se lient à la molécule cible (70) pour former des sondes cadenas circulaires (10') ; et
la détection de l'agglutinat (50) des complexes marqués (40) comprend la détection (S5) de l'agglutinat (50) des complexes marqués (40) pour détecter les molécules d'acide nucléique cibles respectives (1', 1") et ainsi la molécule cible (70).

13. Kit de détection d'au moins une molécule d'acide nucléique cible (1, 1', 1") dans un échantillon, le kit comprenant :
des sondes cadenas (10) comprenant à leurs extrémités 5' et 3' (14, 15) des régions de liaison à la cible (12, 13) complémentaires des régions de liaison à la sonde (2, 3) dans au moins une molécule d'acide nucléique cible (1, 1', 1") ;
des amorces d'amplification marquées (20) comprenant un marqueur détectable (22) et une région de liaison à la sonde (21) complémentaire d'une région de liaison à l'amorce (11) des sondes cadenas (10) ;
des oligonucléotides (30, 30A) comprenant une pluralité de régions de liaison (31) ayant une séquence d'acide nucléique correspondant à au moins une partie des sondes cadenas (10) à l'extérieur de la région de liaison à l'amorce (11) ;
éventuellement une ligase adaptée pour ligaturer les extrémités 5' et 3' (14, 15) des sondes cadenas (10) tandis que les régions de liaison à la cible (12, 13) sont hybridées aux régions de liaison à la sonde (2, 3) pour former des sondes cadenas circulaires (10') ; et
éventuellement une polymérase adaptée à l'amplification en cercle roulant des sondes cadenas circulaires (10') avec les amorces d'amplification marquées (20) pour générer des produits de cercle roulant marqués (20').

14. Puce microfluidique (100) comprenant :
une entrée d'échantillon (110) configurée pour recevoir un échantillon comprenant au moins une molécule d'acide nucléique cible (1, 1', 1")
un canal microfluidique (140) en connexion fluidique avec l'entrée d'échantillon (110) et comprenant :
une section de réactif (141) comprenant i) des sondes cadenas (10) comprenant à leurs extrémités 5' et 3' (14, 15) des régions de liaison à la cible (12, 13) complémentaires des régions de liaison à la sonde (2, 3) dans l'au moins une molécule d'acide nucléique cible (1, 1', 1"), ii) une ligase adaptée pour ligaturer les extrémités 5' et 3' (14, 15) des sondes cadenas (10) tandis que les régions de liaison à la cible (12, 13) sont hybridées aux régions de liaison à la sonde (2, 3) pour former des sondes cadenas circulaires (10'), iii) des amorces d'amplification marquées (20) comprenant un marqueur détectable (22) et une région de liaison à la sonde (21) complémentaire d'une région de liaison à l'amorce (11) des sondes cadenas (10), et iv) une polymérase adaptée pour amplifier en cercle roulant la région circulaire des sondes cadenas (10') avec les amorces d'amplification marquées (20) pour générer des produits de cercle roulant marqués (20') ; et
une section de détection (142) comprenant des oligonucléotides (30, 30A) comprenant une pluralité de régions de liaison (31) ayant une séquence d'acide nucléique correspondant à au moins une partie des sondes cadenas (10) à l'extérieur de la région de liaison à l'amorce (11) ; et
une fenêtre de détection (120, 122, 124) alignée avec au moins une partie de la section de détection (142) et agencée pour permettre un accès visuel à au moins une partie de la section de détection (142).

15. Puce microfluidique selon la revendication 14, dans laquelle
le canal microfluidique (140) comprend également une section de contrôle (143) comprenant des oligonucléotides comprenant une pluralité de régions de liaison ayant une séquence d'acide nucléique complémentaire de la région de liaison à la sonde (21) des amorces d'amplification marquées (20) ; et
la puce microfluidique (100) comprend en outre une fenêtre de contrôle (130) alignée avec la section de contrôle (143) et agencée pour permettre un accès visuel à la section de contrôle (143).
